# FASCICULE DE BREVET EUROPEEN

(11) **EP 0 934 419 B1**
(45) Date de publication et mention de la délivrance du brevet: **05.11.2003**
(21) Numéro de dépôt: 97912254.6
(22) Date de dépôt: 24.10.1997
(51) Int. Cl.: C12N 15/55, C12P 11/00, C12M 1/40

(54) **PROCEDE DE PREPARATION DE L'ACIDE 2-HYDROXY 4-METHYLTHIO BUTYRIQUE PAR UTILISATION D'UNE NITRILASE**
VERFAHREN ZUR HERSTELLUNG VON 2-HYDROXY-4-METHYL-THIOBUTTERSÄURE MIT EINER NITRILASE
METHOD FOR PREPARING 2-HYDROXY 4-METHYLTHIO BUTYRIC ACID USING A NITRILASE

(30) Priorité: 25.10.1996 FR 9613077
(43) Date de publication de la demande: 11.08.1999
(73) Titulaire: AVENTIS ANIMAL NUTRITION S.A., 67300 Schiltigheim (FR)
(72) Inventeur: FAVRE-BULLE, Olivier, F-69003 Lyon (FR); PIERRARD, Jérôme, F-69009 Lyon (FR); DAVID, Christophe, F-69003 Lyon (FR); MOREL, Philippe, F-38200 Chuzelles (FR); HORBEZ, Dominique, F-95130 Franconville (FR)
(74) Mandataire: Guerre, Dominique
(86) Numéro de dépôt international: FR9701913
(87) Numéro de publication internationale: WO98018941

(56) Documents cités:
- EP-A- 0 089 165
- EP-A- 0 206 687
- EP-A- 0 237 620
- EP-A- 0 731 079
- WO-A-91/08287
- WO-A-96/09403
- DATABASE WPI Section Ch, Week 7826 Derwent Publications Ltd., London, GB; Class A97, AN 78-46792A XP002036847 & JP 53 056 385 A (AJINOMOTO KK) , 22 mai 1978
- M. KOBAYASHI ET AL: "Nitrilase in biosynthesis of the plant hormone indole-3-acetic acid....." PROCEEDINGS OF THE NATIONAL ACADEMY OF SCIENCES OF USA, vol. 90, janvier 1993, WASHINGTON US, pages 247-251, XP002036846 cité dans la demande

## Description

La présente invention concerne un nouveau procédé de préparation de l'acide 2-hydroxy 4-(méthylthio) butanoïque (HMTBA) et/ou de son sel d'ammonium (HMTBS).

L'acide 2-hydroxy 4-méthylthio butyrique et ses sels sont utilisés depuis longtemps dans l'alimentation animale en remplacement de la méthionine. Il ou ils présentent l'avantage sur cette dernière de se présenter sous une forme liquide ce qui facilite leur utilisation par les sociétés productrices d'aliments.

Il est connu depuis longtemps de préparer l'acide 2-hydroxy 4-(méthylthio) butanoïque par voie chimique. On peut ainsi citer les brevets EP-142 488, EP-143 000 qui décrivent l'hydrolyse du 2-hydroxy 4-méthylthio hydroxybutyronitrile (HMTBN) par un procédé en deux étapes. La première étape consiste à mettre en contact le 2-hydroxy 4-méthylthio butyronitrile avec un acide minéral fort tel que l'acide chlorhydrique ou sulfurique. Dans une étape ultérieure, après dilution à l'eau, l'hydrolyse est complétée à une température plus élevée. L'acide 2-hydroxy 4-(méthylthio) butanoïque est ensuite extrait avec un solvant organique peu miscible à l'eau tel qu'une cétone, de préférence la méthylisobutyl cétone puis le solvant est éliminé par évaporation. Ce type de procédé, utilisé au niveau industriel, comporte néanmoins quelques inconvénients. Il produit une quantité molaire de sulfate d'ammonium au moins égale à la quantité molaire de nitrile introduite qui doit être éliminé, générant ainsi un déchet industriel allant à l'encontre d'une politique de protection de l'environnement. Ce procédé nécessite aussi l'utilisation de quantités importantes de solvant qu'il est absolument nécessaire de recycler.

D'autres procédés tels que ceux décrits dans les brevets US 3 773 927 et 4 353.924 consistent à hydrolyser le 2-hydroxy 4-méthylthio butyronitrile avec de l'acide chlorhydrique puis à concentrer le milieu avec séparation du chlorure d'ammonium formé. Le sel obtenu est aussi difficile à éliminer que le sel précédent et de plus l'acide obtenu présente une forte coloration.

Il est encore décrit dans le brevet EP 330 521 un procédé d'hydrolyse chimique du méthylthio hydroxypropionitrile qui consiste, comme précédemment, à réaliser une hydrolyse en milieu sulfurique. Le mélange est partiellement neutralisé avec de l'ammoniaque. Une séparation biphasique apparaît. La phase organique contient la majorité de l'acide 2-hydroxy 4-méthylthio butyrique et la phase aqueuse contient la majorité du sulfate d'ammonium produit. La solution organique après évaporation de l'eau contenue est filtrée de façon à récupérer le sulfate d'ammonium dissous. L'acide 2-hydroxy 4-méthylthio butyrique est ensuite dilué avec un peu d'eau et stabilisé avec un peu d'acide sulfurique. La solution aqueuse après élimination de l'eau permet d'obtenir du sulfate d'ammonium directement commercialisable. Ce procédé résoud en partie les inconvénients des procédés de l'art antérieur en ce qui concerne l'usage de solvants organiques mais ne résoud en rien les problèmes liés aux rejets des sels minéraux.

Parmi les brevets relatifs aux sels de l'acide 2-hydroxy 4-méthylthio butyrique, on peut citer les brevets US 2 745 745, 2 938 053 et 3 175 000 concernant les sels de calcium et/ou d'ammonium. Le mélange obtenu par hydrolyse du 2-hydroxy 4-méthylthio butyronitrile est traité avec de l'hydroxyde ou du carbonate de calcium. Le sulfate de calcium est alors précipité libérant l'ammoniaque qui forme le sel d'ammonium de l'acide 2-hydroxy 4-méthylthio butyrique. Ici encore le problème qui consiste à éliminer le sulfate de calcium subsiste.

Il est encore décrit dans la demande de brevet WO 96/01808 un procédé qui consiste à pratiquer une hydrolyse et une-extraction par un solvant organique comme dans le premier document de l'art antérieur cité puis une neutralisation de la solution organique par de l'ammoniaque. Ce procédé, comme la majorité des procédés précédemment décrits, aboutit à la formation d'au moins une mole de sulfate ou de chlorure d'ammonium par mole de nitrile introduite et exige le recyclage de quantités importantes de solvant organique. Il ne peut permettre d'obtenir une solution de sel d'ammonium de l'acide 2-hydroxy 4-méthylthio butyrique à un coût industriellement intéressant.

Il est en outre décrit dans WO96/09403 l'utilisation d'une nitrilase comme catalyseur d'hydrolyse d'un groupe nitrile en groupe carboxylique. Le procédé décrit dans ce document n'est toutefois pas utilisable à l'échelle industrielle, compte tenu de l'activité insuffisante des microorganismes qui synthétisent ces nitrilases.

On a maintenant découvert grâce au procédé de l'invention qu'en mettant en oeuvre plusieurs étapes spécifiques, il était possible d'obtenir l'acide 2-hydroxy 4-méthylthio-butanoïque et/ou son sel d'ammonium par voie enzymatique à l'échelle industrielle, avec un rendement élevé, sans utilisation de solvants et sans production concomitante de sels minéraux.

L'invention a pour objet un procédé de préparation de l'acide 2-hydroxy 4-méthylthio butyrique et/ou du sel d'ammonium de l'acide 2-hydroxy 4-méthylthio butyrique par hydrolyse enzymatique du 2-hydroxy 4-méthylthio butyronitrile caractérisé en ce que :
a) dans une première étape on prépare un matériel biologique ayant une activité nitrilase,
b) dans une deuxième étape, on l'immobilise,
c) dans une troisième étape, on met en présence le 2-hydroxy 4-méthylthio butyronitrile avec le matériel biologique ainsi immobilisé, pour obtenir le sel d'ammonium de l'acide 2-hydroxy 4-méthylthio butyrique,
d) dans une quatrième étape, éventuellement, on convertit le sel obtenu à l'étape c) en l'acide correspondant, et
e) dans une cinquième étape, on concentre le produit obtenu à l'étape c) ou d).

L'invention sera expliquée de façon détaillée dans la description qui suit pour laquelle on se reportera aux figures annexées sur lesquelles :
- la figure 1 représente un schéma d'une cellule d'électrodialyse employée à l'étape facultative d) ; la figuré 1A représente une cellule d'électrodialyse à membrane bipolaire à trois compartiments ; la figure 1B représente une cellule d'électrodialyse à membrane bipolaire à deux compartiments.
- la figure 2 représente un schéma d'une installation pour la mise en oeuvre du procédé selon l'invention ;
- la figure 3 représente la carte de restriction des plasmides pRPA-BCAT1 à 5.
- la figure 4 représente la stratégie de séquençage du fragment de 1130 pb contenant la séquence d'ADN (dénommée sur la figure nitB) codant pour le polypeptide ayant l'activité nitrilase selon l'invention. Les numéros se réfèrent à l'identité des amorces utilisées pour le séquençage ainsi que les notations M13FWD et M13REV.
- la figure 5 représente la carte de restriction des plasmides pRPA-BCAT12 et pRPA-BCAT13.
- la figure 6 représente l'électrophorèse sur gel de SDS-PAGE à 10 % montrant l'expression des séquences d'ADN selon l'invention dans les souches *E. coli* BL21(DE3)/pRPA-BCAT12, BL21(DE3)/pRPA-BCAT13, BL21(DE3)/pRPA-BCAT12 + pXL2231, BL21(DE3)/pRPA-BCAT13 + pXL2231. Chaque piste correspond à une quantité de protéines solubles de 10 µg et un volume équivalent de fraction brute et insoluble.
- la figure 7 représente l'électrophorèse sur gel de SDS-PAGE à 10 % montrant l'expression de la séquence d'ADN de référence selon l'invention dans les souches *E. coli* DH5α/pRPA-BCAT3, DH5α/pRPA-BCAT6, DH5α /pRPA-BCAT6 + pXL2035, RPA-BIOCAT76. Chaque piste correspond à une quantité de protéines solubles de 10 µg et un volume équivalent de fraction brute et insoluble.
- la figure 8 représente la carte de restriction du plasmide pRPA-BCAT14.
- la figure 9 représente l'électrophorèse sur gel SDS-PA à 10 % montrant l'expression des séquences d'ADN selon l'invention dans les souches *P. putida* G2081 (pRPA-BCAT14), G2081 (pRPA-BCAT23), G2081 (pRPA-BCAT24), *A. faecalis* ATCC8750 (pRPA-BCAT14), *A. faecalis* ATCC8750 (pRPA-BCAT23), *A. faecalis* ATCC8750 (pRPA-BCAT24). Chaque piste correspond à une quantité de protéines solubles de 10 µg et un volume équivalent de fraction brute et éventuellement insoluble.
- la figure 10 représente la carte de restriction du plasmide pCGL1087.
- la figure 11 représente la carte de restriction du plasmide pOS48.7.

La première étape consiste à préparer le matériel biologique ayant une activité nitrilase.

Le matériel biologique peut consister en une solution enzymatique telle quelle ou des cellules entières ou brisées ayant une activité nitrilase.

Avantageusement, on utilise un microorganisme exprimant une nitrilase.

La nitrilase peut notamment être issue d'un microorganisme, en particulier un microorganisme du genre *Alcaligenes, Rhodococcus* ou *Gordona,* notamment *Alcaligenes faecalis, Rhodococcus sp*. HT 29-7, *Gordona terrae*, de préférence les souches décrites dans WO 96/09403.

On utilise avantageusement un microorganisme exprimant une activité nitrilase obtenue par transfert de l'information génétique codant pour la nitrilase d'un micro-organisme parental dans un microorganisme hôte. En particulier, chez *E. coli*, on peut utiliser la coexpression des protéines chaperonnes Gro ESL pour améliorer les performances de la souche recombinante.

A cet effet, on utilise tout vecteur d'expression approprié, notamment un vecteur plasmidique.

La séquence nucléotidique utilisée dans ce cas sera alors placée sous le contrôle de signaux permettant son expression dans un hôte cellulaire. L'hôte cellulaire utilisé peut être choisi parmi des systèmes procaryotes, comme les bactéries Gram positif ou Gram négatif ou eucaryotes, comme par exemple les levures, les champignons ou tout autre système. Les signaux contrôlant l'expression des polypeptides sont choisis en fonction de l'hôte cellulaire utilisé. A cet effet, l'ADN codant pour une nitrilase peut être insérée dans des vecteurs à réplication autonome au sein de l'hôte choisi, ou des vecteurs intégratifs de l'hôte choisi. De tels vecteurs seront préparés selon les méthodes couramment utilisées par l'homme du métier, et les .constructions en résultant peuvent être introduites dans un hôte approprié par des méthodes standard telles que l'électroporation.

A titre de microorganisme hôte, on peut citer en particulier *Alcaligenes faecalis, Pseudomonas putida, Escherichia coli*, ou les microorganismes du genre *Bacillus, Corynebacterium, Streptomyces, Saccharomyces, Kluyveromyces, Pénicillium* et *Aspergillus*.

Un vecteur préféré pour l'expression chez *E. coli* est le plasmide pRPA-BCAT6.

La deuxième étape du procédé consiste à immobiliser le matériel biologique. Cette immobilisation se fait avantageusement en présence d'un support solide et permet d'obtenir des particules solides dont la taille, la forme et la résistance mécanique peuvent être contrôlées. Elle permet aussi l'emploi simultané de polymère de polyazétidine et d'autres agents réticulants.

Ce procédé d'immobilisation peut faire intervenir des cellules entières ou perméabilisées. Il peut aussi s'appliquer à une solution enzymatique exempte de cellules.

Les enzymes utilisées pour l'immobilisation sont les nitrilases.

Le procédé d'immobilisation consiste à immobiliser le matériel biologique actif sur un support solide, de granulométrie notamment comprise entre 1 µm et 3 mm, de préférence 10 µm et 2 mm, grâce à des agents chimiques réagissant avec les fonctions amine (NH₂, NH), carboxyle (COOH), hydroxy (OH), thiol (SH) ou amide (CONH₂) de l'agent biologique et du support. Ces agents chimiques permettent aussi d'insolubiliser dans l'eau le matériel biologique et le support. La masse obtenue est très maléable et peut être mise en forme afin d'obtenir des particules de forme et de taille souhaitées. La cohésion et la dureté de ces particules sont ensuite obtenues par séchage.

Le matériel biologique à immobiliser peut éventuellement contenir également un matériel biologique inactif présent à raison de 0 à 200 % en poids. Ce matériel inactif biologique peut être des protéines (albumine, gélatine) ou des polysaccharides (Chitosan, k-carraghénane, alginate).

Le support inerte sur lequel est déposé le matériel biologique et le polymère peut se composer de particules organiques ou inorganiques, poreuses ou non poreuses, hydrophiles ou hydrophobes. Parmi ces particules on peut signaler sans que cela soit limitatif :
- les résines échangeuses d'ions,
- l'alumine,
- les silices de synthèse ou diatomées et les gels de silice,
- les zéolites,
- les charbons,
- les protéines insolubles dans l'eau, comme le gluten,
- les polysaccharides, comme l'amidon.

Le support inerte peut être ajouté à raison de 0,01 à 500% en poids du matériel biologique et de préférence de 10 à 200 %.

Les agents chimiques utilisés pour insolubiliser le matériel biologique peuvent être des polymères ou des molécules bifonctionnalisées qui réagissent avec les fonctions amine (NH₂, NH), carboxylique (COOH), hydroxy (OH), thiol (SH), amide (CONH₂). On peut citer :
- les polymères de polyazétidine,
- les polymères de polyéthylènimine,
- les polymères de polyamide,
- les polymères d'isocyanates,
- les gels d'alginate,
- les gels de k-carraghénane,
- les amines comme l'hexaméthylène diamine,
- les aldéhydes comme le glutaraldéhyde,
- les acides carboxyliques comme l'acide adipique, et
- les isocyanates.

Le procédé d'immobilisation peut faire intervenir un ou plusieurs de ces agents chimiques. L'agent chimique est ajouté selon une concentration comprise entre 1 et 50 % en poids par rapport au matériel biologique et au support. On préférera une quantité comprise entre 5 et 30 % afin d'obtenir des particules suffisament solides et qui conservent une activité importante et qui ne présentent pas trop de problèmes de diffusion interne.

La durée du traitement de réticulation est comprise entre 0,5 et 24 heures.

La température du procédé est généralement comprise entre 4 et 65°C. On préférera une température comprise entre 20 et 40°C. La température employée lors du procédé d'immobilisation peut être aussi très dépendante de la stabilité du matériel biologique employé.

Le pH durant la phase d'immobilisation est maintenu entre 5 et 11. On préférera un pH compris entre 6 et 10 avec une préférence vers les pH alcalins. Le pH est aussi choisi en fonction de la résistance du matériel biologique et sera aisément déterminé par l'homme du métier.

La mise en forme du biocatalyseur doit permettre son emploi dans un système quelconque, notamment dans un lit fixe.

Une méthode de formulation peut être l'extrusion. Pour ce faire, le matériel biologique et le support sont réticulés par ajout d'un ou plusieurs agents chimiques. Après traitement, la masse insoluble est récupérée par centrifugation ou après floculation et filtration. Un taux de matière sèche d'au moins 10% est préféré. La masse est ensuite extrudée. Par cette méthode, on obtient de préférence des vermicelles de diamètre compris entre 0,3 et 0,5 mm et d'une longueur comprise entre 1 et 10 mm. Ces vermicelles peuvent être sphéronisés. Les particules obtenues sont ensuite séchées.

Une autre méthode de formulation peut être le pelliculage ("spray coating"). Pour ce faire, le matériel biologique est mélangé avec un ou plusieurs agents chimiques. Après réaction, le mélange est pulvérisé sur le support sous forme d'une couche mince. Par cette méthode, on obtient des granulés de diamètre moyen compris entre 0,1 et 2 mm.

Les particules obtenues peuvent éventuellement être ensuite plongées dans une solution d'un agent réducteur comme le borohydrure de sodium afin de réduire les fonctions imines formées lors de la réticulation.

Les particules obtenues sont suffisament solides et résistantes à l'attrition pour être employées dans un lit-fixe, un lit fluidisé ou un réacteur agité.

La troisième étape du procédé selon l'invention consiste à mettre en oeuvre le matériel biologique immobilisé dans une ou plusieurs colonnes ou réacteurs. Le but de cette étape est de pouvoir produire en continu le sel d'ammonium de l'acide 2-hydroxy 4-méthylthiobutyrique à partir du 2-hydroxy 4-méthylthiobutyronitrile.

La ou les colonnes ou réacteurs sont alimentées par une solution pure ou diluée du 2-hydroxy 4-méthylthiobutyronitrile ou un mélange contenant du 2-hydroxy 4-méthylthiobutyronitrile et du sel d'ammonium de l'acide 2-hydroxy 4-méthylthiobutyrique.

La ou les colonnes ou réacteurs sont mises en oeuvre de préférence à une température comprise entre 10 et 60°C et à un pH compris entre 5 et 9.

Le système mis en oeuvre peut être constitué de deux ou plusieurs colonnes connectées l'une à l'autre en série, avec selon une première manière de mettre en oeuvre l'invention, l'alimentation de la solution aqueuse du 2-hydroxy 4-méthylthiobutyronitrile en tête de la première colonne avec une alimentation simultanée des autres colonnes avec la solution du 2-hydroxy 4-méthylthiobutyronitrile en une quantité limitée à la solubilité de ce composé dans le mélange réactionnel ; ce système est intitulé système étagé. Selon une deuxième manière de mettre en oeuvre l'invention, on utilise une ou plusieurs colonnes connectées l'une à l'autre en parallèle dans une boucle de circulation. Selon cette installation le 2-hydroxy 4-méthylthiobutyronitrile en solution aqueuse est alimentée en continu dans la boucle, et le milieu de réaction est pompé en continu de façon à conserver un volume constant dans la boucle ; ce système est intitulé système-boucle.

Le type de réacteur utilisé dans cette invention peut être du type lit fixe, lit fluide, ou du type agité en continu. On préfère utiliser les réacteurs du type lit fixe parce qu'ils réduisent les problèmes d'attrition qui peuvent être rencontrés avec les particules de cellules immobilisées. Si le microorganisme est utilisé tel quel, on préférera utiliser un réacteur agité couplé à un module d'ultrafiltration pour séparer de façon continue le microorganisme du produit d'intérêt.

La quatrième étape qui est une étape facultative consiste à convertir le sel d'ammonium de l'acide 2-hydroxy 4-méthylthiobutyrique en l'acide correspondant. Cette étape peut être réalisée suivant deux méthodes :
- soit par électrodialyse au moyen d'un électrodialyseur à deux ou trois compartiments,
- soit par chauffage de la solution aqueuse qui peut être suivi d'une extraction liquide/ liquide.

Suivant la méthode par électrodialyse, on emploiera un électrodialyseur à deux ou trois compartiments.

On entend par "compartiment", l'espace soit entre deux membranes, soit entre une membrane bipolaire et homopolaire, soit entre deux membranes homopolaires adjacentes. On entend par "cellule", un ensemble de deux ou trois compartiments. Un empilement comprend entre 5 et 300 cellules. L'électrodialyseur est composé d'un empilement et comporte bien entendu une anode et une cathode.

Les membranes homopolaires pouvant être utilisées dans le cadre de l'invention se divisent en deux grandes familles, selon leur mode de fabrication.

Ainsi, on peut employer des membranes hétérogènes, préparées à partir de résines échangeuses d'ions, mélangées à un liant tel que le polychlorure de vinyle, le polyéthylène ou autre. L'ensemble ainsi formé peut enduire une trame comme par exemple un tissu de polyester ou de polyacrylonitrile.

On peut utiliser également des membranes homogènes, obtenues par introduction d'un groupement fonctionnel sur un support inerte, par greffage chimique ou radiochimique. La méthode chimique la plus utilisée, consiste généralement à fonctionnaliser un latex d'un polymère comportant des noyaux aromatiques, tel que styrène/divinylbenzène ou styrène/butadiène. Le latex ainsi fonctionnalisé peut servir ensuite à enduire une trame comme pour les membranes hétérogènes. La méthode radiochimique comporte généralement le greffage, sous l'influence d'un rayonnement, d'un composé aromatique, tel que le styrène, sur un support inerte comme une feuille de polyéthylène ou de polytétrafluoroéthylène. Le noyau aromatique est ensuite fonctionnalisé comme dans la méthode chimique.

Les membranes échangeuses de cations comportent des groupements acides forts, le plus souvent des groupements sulfonates, ou des groupements acides faibles, souvent des groupes carboxylates. Plus rarement, les -groupements acides peuvent être des groupements PO₃²⁻, HPO₂⁻, AsO₃²⁻, SeO₃⁻.

Les membranes échangeuses d'anions comportent des groupements basiques forts, le plus souvent des groupements ammonium quaternaire, ou des groupements basiques faibles, le plus souvent des groupements amines. Plus rarement, les groupements basiques peuvent être des groupements phosphonium quaternaire ou des groupements sulfonium.

Dans le présent procédé, les membranes cationiques comportent de préférence des groupements acides forts et parmi ceux ci préférentiellement des groupements sulfonates et les membranes anioniques comportent de préférence des groupements basiques forts et parmi ceux-ci des groupements ammonium quaternaire.

Les membranes bipolaires sont un assemblage de deux membranes, l'une cationique, l'autre anionique. Lorsque la membrane est soumise à un champ électrique suffisant, l'eau de solvatation à l'interface de la membrane se dissocie en ions H⁺ et OH⁻, qui migrent respectivement vers la cathode en traversant la face cationique et vers l'anode en traversant la face anionique. Comme membranes bipolaires, on peut citer à titre d'exemple les membranes commercialisées par les sociétés Aqualytics, Tokuyama Soda ou FuMaTech.

L'anode de l'électrodialyseur peut être constituée par des matériaux classiquement utilisés en électrodialyse, par exemple du graphite, du nickel ou du titane revêtu par des métaux précieux ou des oxydes de métaux précieux, notamment le titane platiné. La cathode peut également constituée par des matériaux classiquement utilisés en électrodialyse, par exemple du graphite, de l'acier inoxydable ou du nickel.

L'électrodialyseur est alimenté avec la solution aqueuse à traiter. Il est également nécessaire de faire circuler à l'anode une solution d'un anolyte et à la cathode une solution d'un catolyte. On peut employer également une solution unique d'électolyte. Dans le présent procédé, un circuit unique d'électrolyte convient bien. Le rôle de la solution d'électrolyte est d'assurer une conductivité suffisante. De préférence, cette conductivité sera égale ou supérieure à 20 millisiemens par centimètre (mS/cm), sans que cette limite inférieure soit à considérer comme critique pour la mise en oeuvre du procédé.

L'électrolyte mis en oeuvre est un composé ionisable tel qu'un sel, un acide ou une base. L'électrolyte est de préférence choisi parmi les composés non électroactifs. Ainsi par exemple, il est préférable d'utiliser des sels neutres comme les sulfates, des acides comme l'acide sulfurique, des bases comme la soude.

Les densités de courant appliquées sont généralement comprises entre 0,2 et 1,5 kA/m², et de préférence entre 0,4 et 1kA/m².

La température à laquelle est mis en oeuvre le procédé de l'invention se situe dans un domaine compatible avec la stabilité des membranes. On opérera de préférence à une température comprise entre 30 et 60°C.

L'électrodialyseur peut fonctionner de différentes façons. Il peut tout d'abord fonctionner en continu, la solution à traiter traversant en continu l'empilement ; plusieurs étages sont alors disposés en série si le taux de traitement à obtenir l'exige. Il peut aussi fonctionner en discontinu, la solution à traiter recirculant sur une cuve jusqu'à ce que le taux de traitement souhaité soit obtenu. Enfin il peut fonctionner en passage direct avec recirculation partielle.

Selon une première variante de l'invention, la dissociation du sel d'ammonium en acide 2-hydroxy-4-(méthylthio)butanoïque et en ammoniaque peut se faire dans une cellule d'électrodialyse à membranes bipolaires à trois compartiments telle que représentée schématiquement à la figure 1A.

Un appareil d'électrodialyse convenable pour la mise en oeuvre du procédé est constitué par différents compartiments, délimités respectivement par des membranes cationiques (MC), des membranes bipolaires (MB) et des membranes anioniques (MA). Ces compartiments se divisent en compartiment sel (S) qui s'appauvrissent en composés à séparer, en compartiment base (B) et acide (A) où se concentrent respectivement l'acide et la base regénérés à partir du sel.

Le sel d'ammonium est introduit dans le compartiment sel. Sous l'action du champ électrique, l'ion ammonium migre vers la cathode en sortant du compartiment (S) où il se trouve, à travers une membrane échangeuse de cations (membrane cationique) et se combine avec les ions OH- provenant de la face anionique de la membrane bipolaire, au sein de laquelle s'effectue la dissociation de l'eau sous l'effet du champ électrique.

Simultanément, les ions carboxylate (2-hydroxy 4-(méthylthio) butanoate) migrent vers l'anode en sortant du compartiment (S) où ils se trouvent, à travers une membrane échangeuse d'anions (membrane anionique). Après passage dans le compartiment (A) suivant, ils sont protonés par l'apport d'ions H+ provenant de la face cationique de la membrane bipolaire. Les trois compartiments (B), (A), (S) adjacents forment une cellule d'électrodialyse.

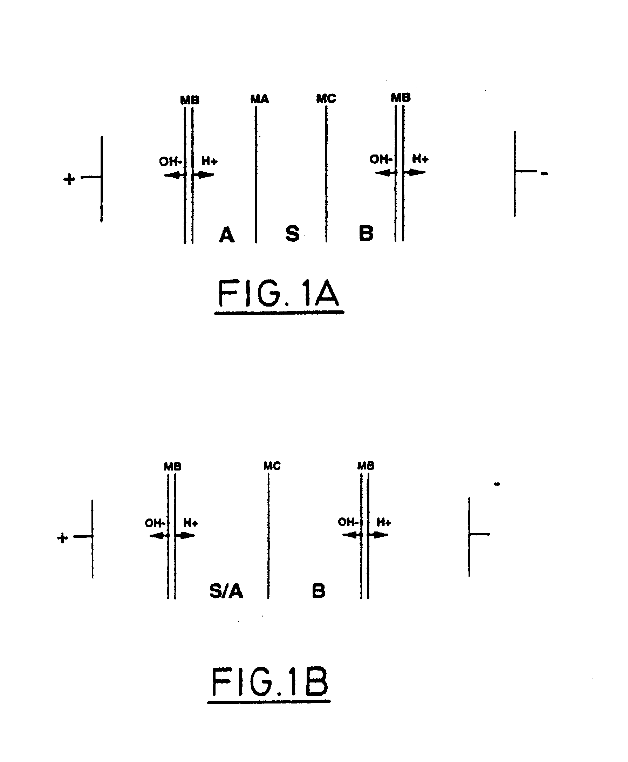

Dans une deuxième variante de l'invention, la régénération de l'acide 2-hydroxy-4-(méhylthio)butanoïque peut se faire dans une cellule d'électrodialyse à membranes bipolaires à deux compartiments telle que représentée à la figure 1B. Ces deux compartiments sont délimités respectivement par des membranes cationiques et des membranes bipolaires. Ces compartiments se divisent en compartiment sel/acide (S/A) et base (B).

Le sel d'ammonium est introduit dans le compartiment sel/acide. Sous l'action du champ électrique, l'ion ammonium migre vers la cathode en sortant du compartiment (S/A) où il se trouve, à travers une membrane échangeuse de cations (membrane cationique) et se combine avec les ions OH- provenant de la face anionique de la membrane bipolaire, au sein de laquelle s'effectue la dissociation de l'eau sous l'effet du champ électrique.

Simultanément, le compartiment S/A s'acidifie par l'apport des ions H⁺ provenant de la face cationique de la membrane bipolaire. Les deux compartiments (B) et (S/A) adjacents forment une cellule d'électrodialyse.

Cette configuration présente l'avantage d'une moindre consommation énergétique et permet de faire varier le rapport sel/acide souhaité.

Pour obtenir un bon fonctionnement de l'électrodyaliseur, la conductivité électrique du compartiment base (de même que celle du compartiment acide dans le cas de la configuration à trois compartiments), doit être suffisante et peut être ajustée par ajout d'un électrolyte support. Ainsi, la conductivité de la solution d'ammoniaque peut être augmentée par ajout d'un sel d'ammonium, comme le sulfate d'ammonium.

Dans une variante préférée de l'invention, on utilisera le 2-hydroxy 4-méthylthio butanoate d'ammonium.

Dans un mode de réalisation particulier envisageable dans le cadre de la présente invention, on introduit dans le compartiment sel d'une cellule d'électrodialyse à trois compartiments un mélange de sel d'ammonium de l'acide 2-hydroxy 4-méthylthiobutyrique et de 2-hydroxy 4-méthylthiobutyronitrile. Le sel sera dissocié comme précédemment en 2-hydroxy 4-méthylthiobutyrate et migrera vers l'anode ou il sera transformé en acide 2-hydroxy 4-méthylthiobutyrique, l'ion ammonium migrera vers la cathode ou il sera transformé en ammoniaque, dans le compartiment sel subsistera l'eau et le 2-hydroxy 4-méthylthiobutyronitrile non transformé qui sera recyclé vers la colonne d'hydrolyse.

Suivant la méthode par chauffage, on récupère l'acide en déplaçant l'équilibre sel d'ammonium, acide libre en chauffant la solution aqueuse riche en HMTBS et en soutirant l'ammoniaque ainsi libérée. On peut y aboutir en chauffant sous vide ou à pression atmosphérique avec ou sans traitement par stripage. L'emploi de CO₂ sous pression peut être envisagé afin de faciliter le déplacement de l'équilibre. On obtient un mélange d'HMTBS et d'HMTBA à raison de 5 à 99,9% d'HMTBA, et de préférence 10 à 50% d'HMTBA par rapport à la somme de l'HMTBA et l'HMTBS. Les viscosité de ces solutions sont comprises entre 1200 et 30 mm².s⁻¹ (1200 et 30 cSt) et de préférence entre 200 et 50 mm².s⁻¹ (200 et 50 cSt) à 25°C.

Les solutions aqueuses peuvent être décomposées par extraction de l'acide avec l'utilisation de produits solvants partiellement miscibles ou non à l'eau. Il existe un grand nombre de solvants possibles pour la séparation. Les solvants préférés sont les cétones de C5 à C8, en particulier la méthylisobutylcétone; les éthers comme l'isopropyléther; les alcools comme isopropanol; les esters; les amines tertiaires comme la trioctylamine. Dans le cadre de l'invention, les solvants préférés sont: l'acétone, la MIBK, l'isoprapanol, l'acétate d'isopropyle, l'éther isobutylique ou propylique ou le THF, la trioctyl amine. Le rapport de la phase aqueuse et de la phase organique n'est pas critique. Cependant, pour des raisons de degré d'efficacité, on ne doit pas descendre en-dessous d'un rapport minimum de 1/1 et pour des raisons de rentabilité ne pas aller au delà d'un rapport de 1/3. On préférera un rapport 1/1,5 à 2,0.

L'extraction peut être mise en oeuvre en batch ou en continu dans n'importe quel type de technologie d'extraction liquide-liquide. On peut citer par exemple la cascade de mélangeurs décanteurs à co- ou contre courant. les extracteurs centrifuges, les colonnes garnies ou à plateaux, etc.

La solution aqueuse appauvrie en acide libre peut être de nouveau traitée et ce, autant de fois que souhaité jusqu'à complet épuisement de l'ammoniaque si désiré.

La phase organique est soumise à un traitement pour isoler l'HMTBA. Ceci est réalisé de préférence par vaporisation du solvant ou par une extraction à l'eau chaude. Pour éviter une éventuelle détérioration de l'HMTBA, on pourra maintenir la sollicitation thermique aussi faible que possible par application d'un vide.

Dans ces deux procédés de dissociation du sel d'ammonium, on génère une solution aqueuse d'ammoniaque. Cette dernière peut être traitée afin de concentrer l'ammoniaque. On préférera la distillation et la concentration de l'ammoniaque en un ou plusieurs étages avec ou sans pression. Une étape préliminaire de stripage peut être envisagée. La solution concentrée d'ammoniaque peut être retournée dans la synthèse de l'acide cyanhydrique qui rentre en jeu dans la synthèse du 2-hydroxy 4-méthylthio butyronitrile.

Dans la cinquième étape, on concentre la solution aqueuse d'HMTBS et/ou d'acide libre. Ceci est réalisé de préférence par évaporation de l'eau.

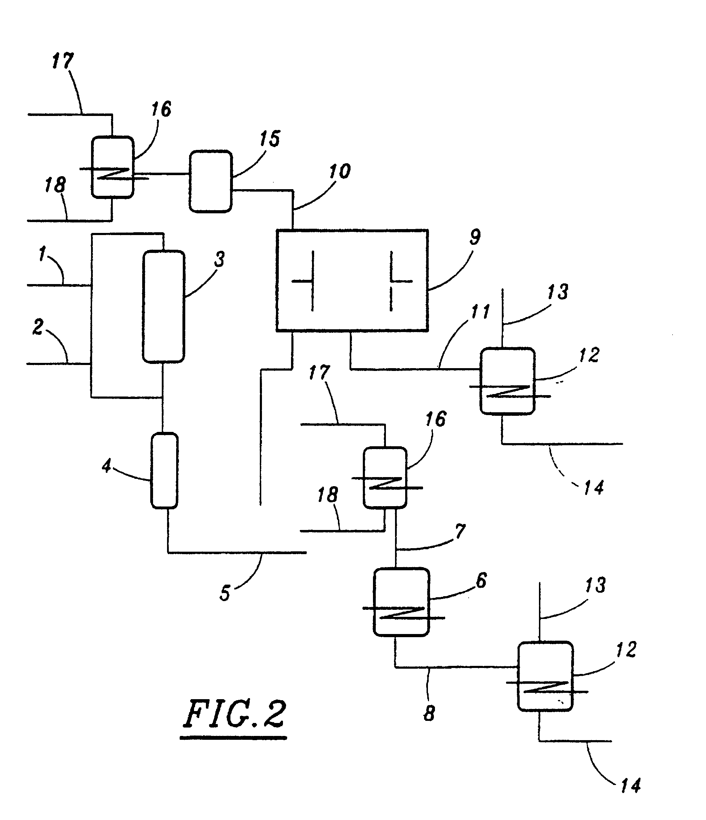

L'invention a également pour objet une installation pour la mise en oeuvre du procédé selon l'invention. Une telle installation est illustrée schématiquement à la figure 2 et comprend des conduites 1,2 destinées à introduire respectivement la cyanhydrine de l'AMTP et l'eau dans le réacteur 3. Le réacteur 3 est un lit fixe à recirculation qui est garni avec les souches ou enzymes immobilisées. Une partie de la solution est soutirée du réacteur 3 et envoyée sur une réacteur finisseur 4. Le réacteur finisseur 4 est de type lit fixe garni avec les souches ou enzymes immobilisées. Une solution concentrée en HMTBS est ensuite récupérée en sortie du réacteur 4 via la conduite 5.

Cette solution concentrée en HMTBS peut subir deux traitements totalements indépendants suivant le type de produit final souhaité.

Si l'on souhaite récupérer un produit contenant une forte proportion d'HMTBS, la solution de la conduite 5 est amenée dans un évaporateur 6 qui permet de concentrer le produit et l'on récupère dans la conduite 8 une solution concentrée composée principalement d'HMTBS et contenant un peu d'acide libre. L'eau en excès ainsi qu'une fraction de l'ammoniaque sont évacuées par la conduite 7.

Si l'on souhaite récupérer un produit contenant une forte proportion d'acide libre, la solution concentrée d'HMTBS de la conduite 5 est amenée dans un système d'éJectrodialyse bipolaire 9. Dans cet appareil, l'ammoniaque est plus ou moins séparée de l'acide par électrodialyse. Le mélange appauvri en ammonium est récupéré dans la conduite 11 puis concentré dans l'évaporateur 12 afin d'obtenir une solution concentrée composée principalement d'acide libre et contenant peu ou pas d'HMTBS. La solution riche en ammoniaque est soutirée par la conduite 10. L'ammoniaque est récupérée par un stripage 15.

Cet effluent d'ammoniaque peut être concentré par distillation 16, l'eau est récupérée en 18. La solution d'ammoniaque concentrée 17 peut être retournée à la synthèse de l'HCN.
Les exemples suivants illustrent l'invention.

### EXEMPLES

### Exemple 1 : Purification et séquençage Nter de la nitrilase

La nitrilase a été purifiée en quatre étapes. Le résumé de la purification est donné dans le tableau 1.

Les cellules de *Alcaligenes faecalis* sont cultivées 24 heures, à 30°C, dans un milieu minimum en présence de benzonitrile (0,5 g/ l). Après centrifugation de la culture, le culot est repris dans du tampon TG (25 mM Tris-HCI, 10% (p/ v) glycérol, pH 7,5). La suspension cellulaire est traitée aux ultrasons puis centrifugée afin d'obtenir l'extrait brut. L'extrait brut est ensuite traité avec du sulfate d'ammonium jusqu'à 30% de la saturation. Le précipité obtenu est resuspendu dans du tampon TG puis dialysé contre 2 litres du même tampon durant une nuit. La solution obtenue est ensuite déposée sur une colonne échangeuse d'anions Q Sepharose Fast Flow HR 26/ 10 préalablement équilibrée avec du tampon TG. L'activité est ensuite éluée avec un gradient de 0 à 1 M NaCI. Les fractions actives sont ensuite déposées sur une colonne échangeuse d'anions Mono Q HR 5/5 préalablement équilibrée avec du tampon TG. La nitrilase est éluée à l'aide d'un gradient de 0 à 1 M NaCl. Pour finir, les fractions contenant l'activité sont réunies, la concentration en sulfate d'ammonium est ensuite amenée à 1 M. Cette solution est alors déposée sur une colonne d'interactions hydrophobes Phényl Superose HR 5/ 5 préalablement équilibrée avec du tampon TG additionné de 1 M (NH₄)₂SO₄. L'activité est ensuite éluée par un gradient de 1M à 0M en sulfate d'ammonium.

**Tableau 1 :**

| *Résumé de la purification de la nitrilase* | | | | | |
|---|---|---|---|---|---|
| Fraction | Prot totale (Mg) | Activité totale (µMol/h) | Activité spécifique (µmole/h.mg) | Rdt en protéine (%) | Facteur de purification |
| Cellules entières | 955 | 4300 | 4,5 | 100 | 1 |
| Extrait brut | | 2400 | | | |
| Sulfate d'ammo. | | 650 | | | |
| QSHL 26/10 | 3 | 360 | 120 | 0.3 | 27 |
| MonoQ HR5/5 | 1,5 | 240 | 160 | 0,15 | 35 |
| Phényl sépharose | 1 | 120 | 110 | 0,1 | 24 |

***Conditions opératoires:*** *[nitrile] = 50 mM; tampon phosphate 100 mM pH 7,0; 30°C*.

Le poids moléculaire de la protéine est déterminé par gel filtration. Il est d'environ 260 kDa. Sur gel de SDS-PAGE, on observe une unique bande de 43 kDa (pureté de 95%). C'est donc probablement une protéine de structure α₆ avec α pesant 43 kDa.

### Exemple 2: Clonage de la nitrilase d'Alcaligenes faecalis ATCC8750

La séquence NH2 terminale présentée dans l'exemple 1 présente une identité totale avec la séquence de l'extrémité N-terminale de la nitrilase d'*A*. *faecalis* JM3 (Kobayashi *et al*., 1993, *Proc. Natl. Acad. Sci. USA* 90: 247-251), alors que les extrémités N-terminales des nitrilases bactériennes présentent de 35 à 57% d'identité sur 14 résidus. Les inventeurs ont émis l'hypothèse que la nitrilase de l'invention purifiée à partir de la souche ATCC8750 était semblable à celle décrite par Kobayashi *et al*. (précité). La stratégie de clonage a alors consisté à amplifier par réaction de PCR sur l'ADN génomique de la souche ATCC8750 le gène de cette nitrilase, à l'aide de deux sondes nucléotidiques determinées à partir de la séquence donnée par Kobayashi *et al*. (précité).

Les deux sondes ont été synthétisées, l'une pouvant s'hybrider avec la partie 5' de la séquence donnée par Kobayashi *et al*. (précité) et l'autre avec la partie 3' :
Partie 5' (PCRAF1): CCGGGAATTCATATGCAGACAAGAAAAATCGTCC
Partie 3' (PCRAF2): TCCTTCTGCGTCCCCGATCCCGCAT

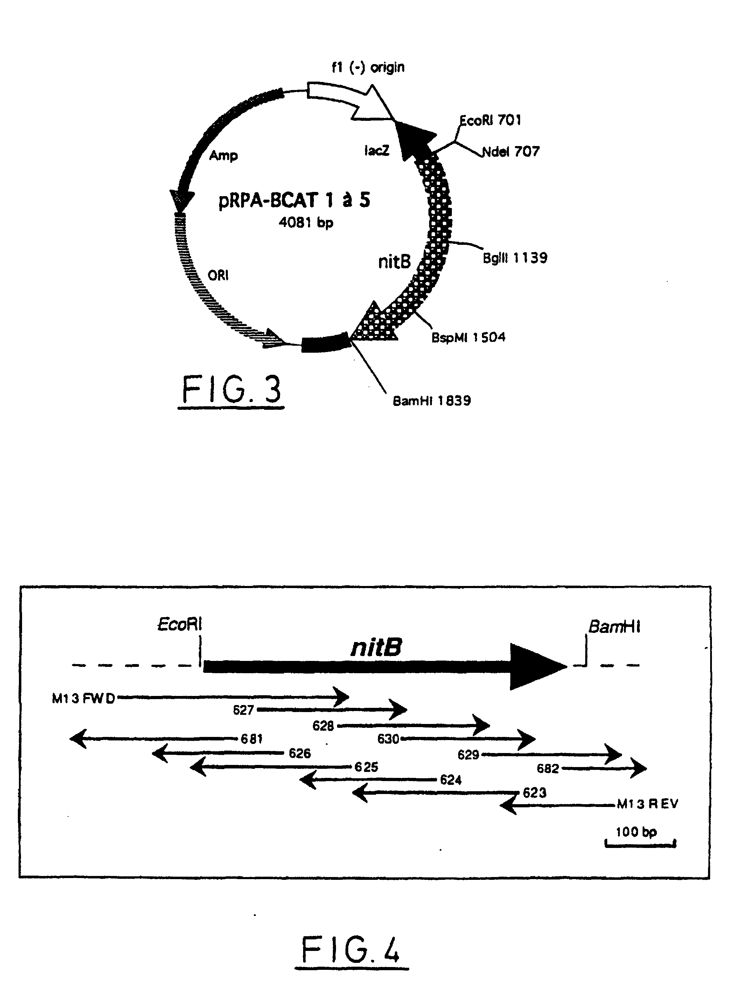

L'amorce PCRAF1 comporte 12 nucléotides en 5' permettant d'introduire en amont du codon d'initiation ATG les sites de restriction des enzymes *Eco*RI et *Nde*I. L'amorce PCRAF2 permet d'introduire le site de restriction de l'enzyme *Bam*HI. L'ADN génomique de la souche d'*A. faecalis* ATCC8750 a été extrait selon le protocole CTAB décrit par Ausubel *et al*. (Current Protocols in Molecular Biology, John Willey & Sons Inc. Ed, 2.4.1-2.4.5) et 100 ng ont été utilisé pour chaque réaction de PCR. Afin de jouer sur la spécificité d'amplification, différentes concentrations en MgCl₂ ont été testées comme indiqué dans Ausubel *et al*. (précité) dans un volume total d'échantillon de 100 µl et avec 2,5 unités de Taq DNA polymerase (Perkin Elmer). Deux réactions. supplémentaires ont été réalisées avec 1,5 mM MgCl₂ et 5% DMSO ou 5% formamide. Un thermocycleur Perkin Elmer 9600 a été programmé avec l'enchaînement suivant: 5 min à 95°C, 30 cycles (30 sec à 95°C, 30 sec à 55°C, 45 sec à 72°C) et.4 min à 72°C. Les différents produits d'amplification ont été analysés sur gel d'agarose 0,8%. Une bande majoritaire, dont la taille correspond à la taille attendue de 1,15 kb, a été amplifiée dans toutes les réactions mais plus spécifiquement avec 1,5 mM MgCl₂ et 5% DMSO. Les produits de la réaction dans ces conditions ont donc été retenus pour la suite. L'échantillon a été traité à la protéinase K, précipité à l'éthanol après une extraction phénol-chloroforme. Le culot repris en suspension a été incubé pendant 2h à 37°C avec 40 unités d'enzyme *Eco*RI et 40 unités d'enzyme *Bam*H1. Après migration sur gel d'agarose 0,7 %, la bande de 1,15 kb a été découpée et extraite pour être clonée dans le vecteur pBSK- (Stratagène, La Jolla, USA) ouvert *Eco*RI-*Bam*HI par les méthodes classiques. Cinq clones indépendants, appelés pRPA-BCAT1 à 5, ont été analysés par digestion enzymatique de l'ADN plasmidique avec les enzymes *Nde*I, *Eco*RI, *Bam*HI, *Bsp*MI et *Bgl*II (figure **3**). Les profils obtenus correspondaient aux profils de restriction théorique d'un plasmide obtenu par cette méthode avec la séquence décrite par Kobayashi *et al*. (précité). La souche XL1Blue (pRPA-BCAT3) a été déposée à la CBS sous le numéro CBS 998-96.

### Exemple 3 : Séquençage d'un fragment de 1130 pb contenant l'ADN codant pour le polypeptide ayant l'activité nitrilase.

L'insert cloné dans le plasmide pRPA-BCAT3 a été séquencé par la société Génome Express S.A. (Grenoble, France) à partir d'une préparation d'ADN faite au laboratoire (kit Wizzard Midi-prep, Promega). La stratégie de séquençage de ce fragment, réalisé selon des méthodes classiques connues de l'homme de métier, est indiquée dans la figure 4. Les dix amorces nucléotidiques internes (identifiées par les numéros 623 à 629 et 681 à 682 sur la figure 4) ont été synthétisées d'après la séquence de la nitrilase d'*A*. *taecalis* JM3 (Kobayashi *et al*., précité). Cet ensemble a été complété par les amorces universelles "Reverse" et "M13 Forward". Chaque région a été lue au moins une fois sur chaque brin de l'ADN.

La séquence d'ADN obtenue présente deux différences par rapport à la séquence publiée : l'une dans la structure supposée servir de terminateur de transcription et l'autre dans le gène de la nitrilase, appelé *nitB*, conduisant à la substitution Asn²⁷⁹-->Asp. Ces deux régions ont alors été séquencées au laboratoire sur les plasmides pRPA-BCAT1, 2, 4, 5 avec deux amorces spécifiques 710 et 682 (cf. figure **4**). Le changement C->T à la position 1412 (conformément à la numérotation de Kobayashi, précité) dans le terminateur a été retrouvée dans tous les clones. Il s'agit d'une mutation qui différencie les deux souches d'*A. faecalis*. Le changement A->G à la position 1138 n'est présent que dans le plasmide pRPA-BCAT3. Il s'agit donc d'une mutation introduite lors de la PCR par la Taq DNA Polymerase.

### Exemple 4 : Expression de la nitrilase dans E. coli BL21 (DE3).

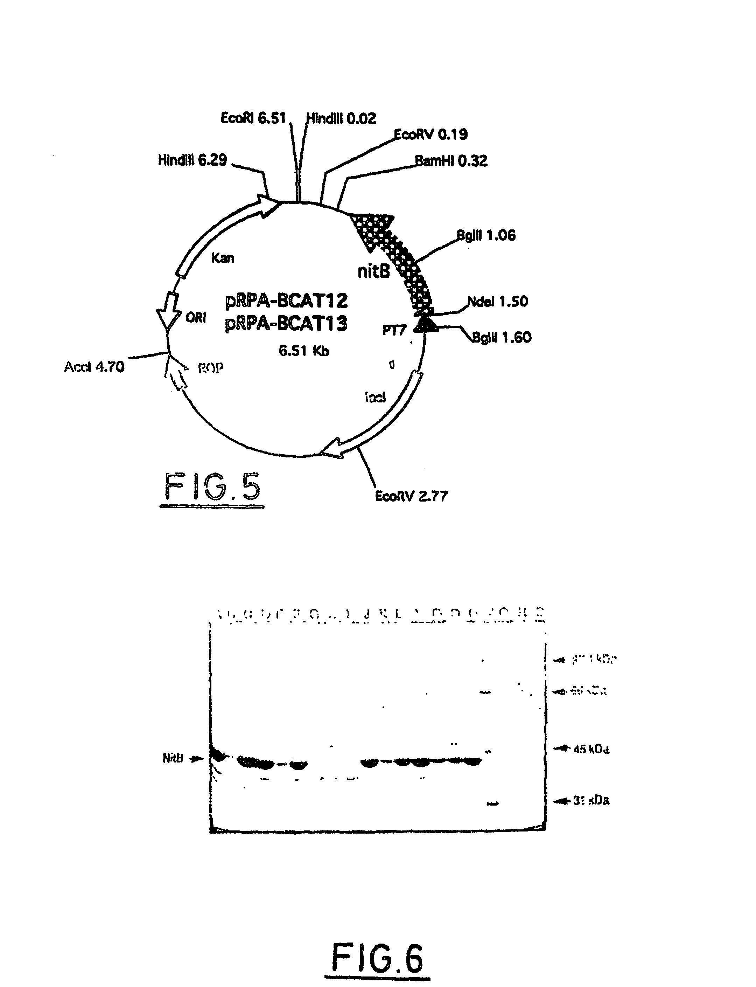

Afin de confirmer l'identification de la séquence d'ADN clonée avec le gène de la nitrilase purifiée, le gène *nit*B a été placé sous le contrôle du promoteur du gène Φ10 phage T7 (PT7) selon le mode opératoire décrit ci-après: les inserts *Nde*I*-Bam*HI de 1,13-kb des plasmides pRPA-BCAT3 et pRPA-BCAT4 ont été clonés dans le vecteur pXL2432 pour donner respectivement les vecteurs pRPA-BCAT12 et pRPA-BCAT 13 décrits sur la figure **5**. Le vecteur parent pXL2432 est un hybride entre la région du plasmide pET9 (Studier *et al*., 1990, Methods In Enzymol. 185, 60-89), comprise entre les sites *Acc*I et *Eco*RI, qui englobe l'origine de réplication (ORI) et le marqueur de sélection porteur de la résistance à la kanamycine (Kan), et la région comprise entre les sites *Eco*RI et *Acc*I du plasmide pET11a (Novagen Inc., Madison Wl, USA) qui englobe la cassette d'expression , le gène du répresseur lacl et le gène du régulateur du nombre de copies ROP.

Des cultures ont été menées en condition d'induction selon le mode opératoire ci-après: La souche BL21(DE3) (Novagen Inc., Madison Wl, USA) contenant le plasmide pRPA-BCAT12, la souche BL21(DE3) contenant le plasmide pRPA-BCAT13 ainsi que la souche BL21(DE3) contenant le plasmide pXL2432 ont été cultivées 16 h en milieu LB à 37°C (Miller, 1972, Experiments in Molecular Genetics - Cold Spring Harbor Laboratory, Cold Spring Harbor, N. Y.) contenant 50 µg/ml de kanamycine, puis diluées au 100ème dans le même milieu et à la même température. Lorsque les cultures ont atteint une DO600 comprise entre 0,5 et 1, de l'IPTG, à la concentration finale de 1 mM a été ajouté. Après 6 heures de culture, les bactéries ont été collectées. Un mode opératoire similaire a été adopté pour cultiver la souche BL21(DE3) contenant les plasmides pRPA-BCAT12 et pXL2231, la souche BL21(DE3) contenant les plasmides pRPA-BCAT13 et pXL2231 ainsi que la souche BL21(DE3) contenant les plasmides pXL2432 et pXL2231 en ajoutant au milieu de culture de la tétracycline à raison de 12 µg/ml de milieu. Le plasmide pXL2231, qui dérive du vecteur pXL1635 (Demande FR 90/05185 du 24/04/1990), appartient au groupe d'incompatibilité IncP et est donc compatible avec les plasmides pRPA-BCAT12 et 13 qui possèdent l'origine de réplication du plasmide ColE1. Son marqueur de sélection est la résistance à la tétracycline et il porte un fragment *Eco*RI-*Hin*dIII de 2,2 kb contenant les gènes GroES et GroEL codant pour des chaperons moléculaires d'*E. coli* (Fayet *et al*., 1986, Mol. Gen. Genet. 202: 435-445).

L'expression de la nitrilase a été analysée en gel 10 % SDS-PA dans la fraction brute après sonication des cellules, et après centrifugation dans le culot et dans le surnageant. Les résultats sont présentés sur la figure 6 et montrent un niveau élevé d'expression de la nitrilase (NitB) dans les extraits de cellules dont un des plasmides au moins contient l'insert *nitB* ; toutefois, cette protéine est essentiellement sous forme insoluble bien que la présence du plasmide pXL2231 permette d'augmenter la quantité de polypeptide nitrilasique dans la fraction soluble.

Sur la figure 6, M représente le marqueur de poids moléculaires ; ceux-ci sont indiqués en kDa. D'autre part, les pistes ont les significations ci-après :
- A, D, G représentent les fractions brutes respectivement de BL21 (DE3) + p/RPA-BCAT12/RPA-BCAT13/XL2432 ;
- B, E, H représentent les surnageants obtenus respectivement à partir de ces mêmes souches ;
- C, F, I représentent les culots obtenus respectivement à partir de ces mêmes souches ;
- J, N, Q représentent les fractions brutes obtenues respectivement à partir de
   BL21(DE3) + pXL2231 + p/RPA-BCAT12/RPA-BCAT13/XL2432 :

- K, O, R représentent les surnageants obtenus respectivement à partir des ces souches ;
- L, P, S représentent les culots obtenus respectivement à partir de ces souches.

La souche BL21(DE3) / pRPA-BCAT12 + pXL2231 a été appelée RPA-BIOCAT126. La souche BL21(DE3) / pRPA-BCAT13 + pXL2231 a été appelée RPA-BIOCAT127. Les activité nitrilasiques de cultures de RPA-BIOCAT126, RPA-BIOCAT127, et RPA-BIOCAT66 qui correspond à BL21(DE3) / pXL2432 ont été determinées comme suit : les cultures ont été menées selon le protocole décrit ci-dessus en modifiant le volume de culture (50 ml) et la concentration finale d'IPTG (0,1 mM). Les cultures ont été centrifugées et les culots cellulaires repris dans 10 ml de tampon phosphate de potassium 100 mM pH 7. L'hydrolyse d'HMTBN est réalisée avec 500 µl de cette suspension ajoutée à 500 µl de solution d'HMTBN 200 mM pH7. Une cinétique d'hydrolyse est obtenue en mélangeant 100 µl de ce mélange à 900 µl d'acide phosphorique 0,1 N à intervalles réguliers pendant 1 à 4 heures. Les quantités d'HMTBA produite sont analysées par HPLC comme décrit dans la demande WO 96/09403. Les résultats sont regroupés dans le tableau 2.

**Tableau 2 :**

| *Activités des souches RPA-BIOCAT66, 126 et 127* | | | | | |
|---|---|---|---|---|---|
| **RPA-BIOCAT** | **MILIEU** | **INDUCTION** | **TEMPS DE CULTURE** | **OD660** | **ACTIVITE (U)** |
| **66** | LB Km | 0,1 mM IPTG | 24 h | 2,5 | 0 |
| **126** | LB Km Tc | 0,1 mM IPTG | 24 h | 2,4 | 15 |
| **127** | LB Km Tc | 0,1 mM IPTG | 24 h | 1,9 | 10 |
| ABREVIATIONS : Km : Kanamycine 50 µg/ml; Tc : tétracycline 12 µg/ml; h : heures; U : kg d'HMTBA formé par heure et par kg de poids sec. | | | | | |

Afin d'améliorer-la solubilisation du polypeptide nitrilasique exprimé, le plasmide pRPA-BCAT37 a été construit comme ci après. Le plasmide pXL2391 a été obtenu en ligaturant le fragment de 5,9 kb *Eco*RI*-Pvu*II du plasmide pDSK519 (Keen *et al*., 1988, Gene 70: 191-197), traité avec la polymérase Klenow, avec le fragment *Hin*dIII de 2056 bp extrait du plasmide pHP45ΩSp (Prentki et Krisch, 1984, Gene 29: 303-313) traité à la Nucléase Mung Bean. Le plasmide pXL2391 a ensuite été digéré par *Sma*I et *Sac*I et l'insert de 2,3 kb portant l'opéron GroESL, extrait du plasmide pXL2231 digéré par *Hin*dIII, traité à la Klenow et digéré par *Sac*I, y a été introduit. Le plasmide pRPA-BCAT37 est donc un dérivé du plasmide RSF1010 à plus fort nombre de copies que le plasmide pXL2231, compatible avec les plasmides porteurs de l'origine ColE1 et portant un marqueur de resistance à la streptomycine. Ce plasmide a été introduit dans la souche BL21(DE3) / pRPA-BCAT12 pour donner la souche RPA-BIOCAT171. L'activité d'une culture menée selon un mode opératoire similaire à celui décrit ci-dessus, mais en remplaçant la tétracycline par la streptomycine à 100 µg /ml, a été déterminée et est rapportée dans le tableau 3.

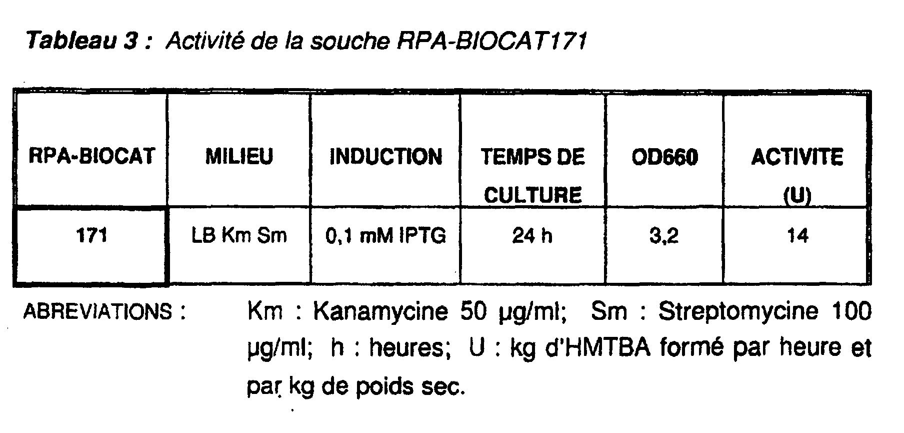

### Exemple 5: Expression de la nitrilase dans E. coli DH5alpha.

Le plasmide pRPA-BCAT6 a été construit en clonant sur pBCAT3 (cf. figure 3) le fragment de 0,6 kb *Scal-Ndel* de pXL2158 contenant le promoteur P*trp* et le site de fixation du ribosome RBScII (Levy-Schill *et al*., 1995, Gene 161: 15-20). L'expression a été réalisée avec la souche DH5alpha contenant le plasmide pRPA-BCAT6 et/ou le plasmide pXL2035 (Levy-Schill *et al*., précité) avec le mode opératoire suivant : la préculture est incubée 16h à 37°C en milieu M9 glucose (Miller, J. H. 1972. Experiments in Molecular Genetics. Cold Spring Harbor Laboratory, Cold Spring Harbor, N.Y.) contenant 0,4% de casaminoacides, 100 µg/ml de tryptophane, 100 µg/ml de carbenicilline. Pour les souches contenant pXL2035, de la kanamycine est ajoutée à raison de 50 mg/l. L'expression se fait après dilution au 1/100 de la culture saturée dans un milieu identique mais sans tryptophane et incubation 8 à 16 h à 37°C.

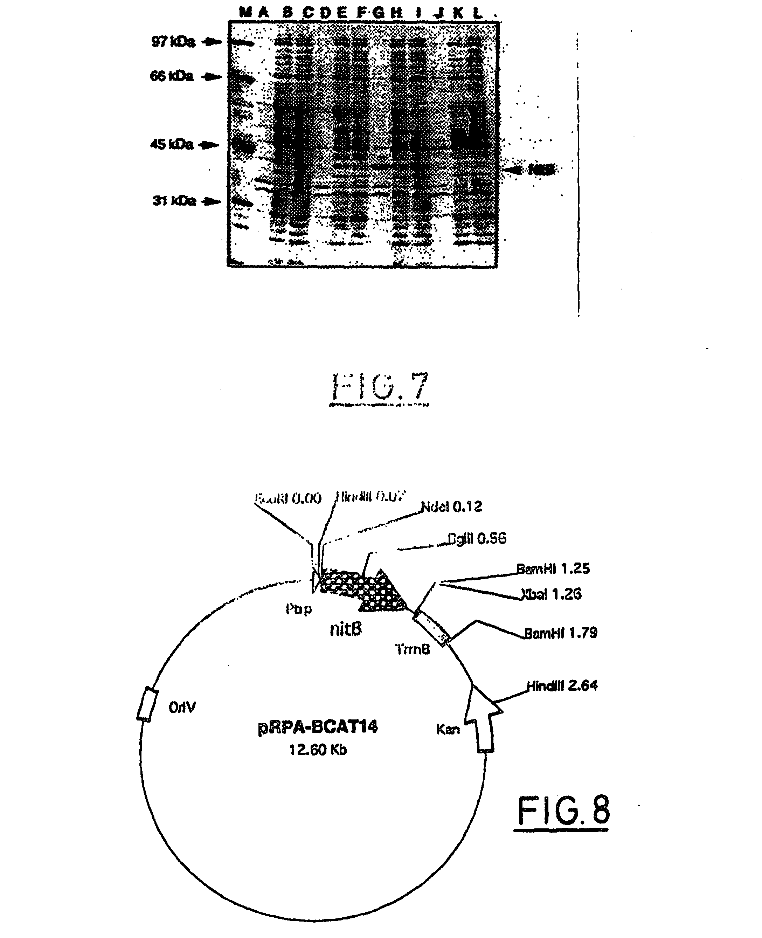

L'analyse en SDS-PAGE des extraits des différentes souches est réalisé comme décrit dans l'exemple précédent et est présentée sur la figure 7.

Sur cette figure, M représente le marqueur de poids moléculaires ; ceux-ci sont indiqués en kDa. D'autre part, les pistes ont les significations ci-après :
- L, I, F, C représentent les fractions brutes obtenues respectivement à partir des souches DH5alpha + pRPA-BCAT/3, /6, /6 + pXL2035, RPA-BIOCAT76 ;
- K, H, E, B représentent les surnageants obtenus respectivement à partir de ces mêmes souches ;
- J, G, D, A représentent les culots obtenus respectivement à partir de ces mêmes souches.

Le plasmide pRPA-BCAT6 permet d'obtenir une faible accumulation d'un polypeptide à 43 kDa, majoritairement insoluble. La coexpression de GroE à partir du plasmide pXL2035 réduit la surexpression, mais après trois repiquages successifs de la souche DH5alpha (pRPA-BCAT6 + pXL2035), la souche RPA-BIOCAT76 sélectionnée retrouve un niveau initial d'expression du polypeptide nitrilasique, celui-ci étant quasi complètement soluble. Les dosages d'activités des cultures réalisés selon les modes opératoire décrits ci-dessus et dans l'exemple précédent sont présentés dans le tableau 4.

**Tableau 4:**

| *Activité des souches DH5alpha (pRPA-BCAT3), DH5alpha (pRPA-BCAT6), DH5alpha (pRPA-BCAT6 + pXL2035), RPA-BIOCAT76* | | | | |
|---|---|---|---|---|
| **SOUCHE** | **MILIEU** | **TEMPS DE CULTURE** | **OD660** | **ACTIVITE (U)** |
| **DH5alpha (pRPA-BCAT3)** | M9 Cb | **24** h | 4 | 0 |
| **DH5alpha (pRPA-BCAT6)** | M9 Cb | **24** h | 4 | 0,6 |
| **DH5alpha (pRPA-BCAT6 + pXL 2035)** | M9 Cb Km | **24** h | 3 | 1,6 |
| **RPA-BIOCAT76** | M9 Cb Km | **24** h | 3,8 | 5 |
| **ABREVIATIONS :** Km : Kanamycine 50 µg/ml; Cb : Carbenicilline 100 µg/ml; h : heures; U : kg d'HMTBA formé par heure et par kg de poids sec. | | | | |

Ces données montrent que la coexpression de GroE permet d'améliorer l'expression de la nitrilase et qu'une sélection de souche classique par repiquages successifs contribue aussi à l'amélioration de l'activité des recombinants.

### Exemple 6 : Expression de la nitrilase dans Pseudomonas putida et Alcaligenes faecalis.

Le système d'expression décrit dans l'exemple 5 a été utilisé pour produire la nitrilase chez *Pseudomonas putida* et *A. faecalis.* Le fragment de 1,14 kb *Nde*I*-Xba*I de pRPA-BCAT6 contenant le gène *nitB a* été introduit dans les sites *Nde*I-*Xba*I du vecteur pXL1289. Le vecteur pXL1289 est un dérivé de pKT230 (Bagdasarian *et al*., 1981, Gene 15: 237-247) portant l'origine de réplication (oriV) multi-hôte chez les bactéries Gram-négatives et qui contient un fragment *EcoR*I*-Nde*I de 120 bp porteur du promoteur P_{*trp*} et du RBScII de pXL534 (Latta *et al*., 1990, DNA and Cell Biology, 9: 129-137), un insert *Nde*I*-Xba*I codant pour le gène *cobA* (Crouzet *et ai.,* 1990, J. Bacteriol. 172: 5968-5979) et un insert *Xba*I*-Bam*HI de 500 bp contenant le terminateur T_{*rrnB*} de pXL534 (Latta *et al*., 1990, DNA and Cell Biology, 9: 129-137). Le plasmide obtenu pRPA-BCAT14 est donc un dérivé de pKT230 contenant un gène conférant la résistance à la kanamycine (Kan) et le gène *nitB* sous le contrôle de P*trp*:RBScll (figure **8**).

Lors de l'introduction de ce plasmide dans la souche d'*E. coli* DH5alpha, un clone particulier a été sélectionné : il exprimait une nitrilase dont le poids moléculaire sur gel SDS-PA est de 44 kDa au lieu de 43 kDa. Le plasmide hébergé par ce clone présente le même profil de restriction que pRPA-BCAT14 et a été appelé pRPA-BCAT24. Enfin, le plasmide pRPA-BCAT23 a été construit selon le même mode opératoire que pRPA-BCAT14 avec la modification suivante : le fragment de 136 bp *Stu*I-*Bsm*I de pRPA-BCAT6 a été remplacé par le fragment *Stu*I-*Bsm*I de pRPA-BCAT4. Le plasmide pRPA-BCAT23 exprime donc une nitrilase NitB avec un résidu Asn à la position 279.

Les plasmides pRPA-BCAT14, 23 et 24 ont été introduits par électroporation dans la souche *Pseudomonas putida* G2081. La souche G2081 dérive de la souche KT2440 (Bagdasarian and Timmis, 1981, in Hofschneid and Goebel, Topics in Microbiology and Immunology, 47, Springer Verlag, Berlin) par sélection de la résistance spontanée à l'acide nalidixique et à la rifampycine. Le vecteur pKT230 a été utilisé comme plasmide contrôle.

Les plasmides pRPA-BCAT14, pRPA-BCAT23, pRPA-BCAT24 et pKT230 ont alors été extraits des souches de *P. putida* pour être introduits par électroporation dans la souche d'*A*. *faecalis* ATCC8750 (= RPA-BIOCAT1).

Les souches G2081 (pRPA-BCAT14), G2081 (pRPA-BCAT23), G2081 (pRPA-BCAT24), G2081 (pKT230) et RPA-BIOCAT1 (pRPA-BCAT14), RPA-BIOCAT1 (pRPA-BCAT23), RPA-BIOCAT1 (pRPA-BCAT24), RPA-BIOCAT1 (pKT230) ont été cultivées la nuit à 30°C dans du milieu LB contenant 50 mg/l de kanamycine. Ces précultures ont été diluées au 1/100 dans du milieu M9 contenant 50 mg/l de kanamycine et incubées 20 h à 30°C.

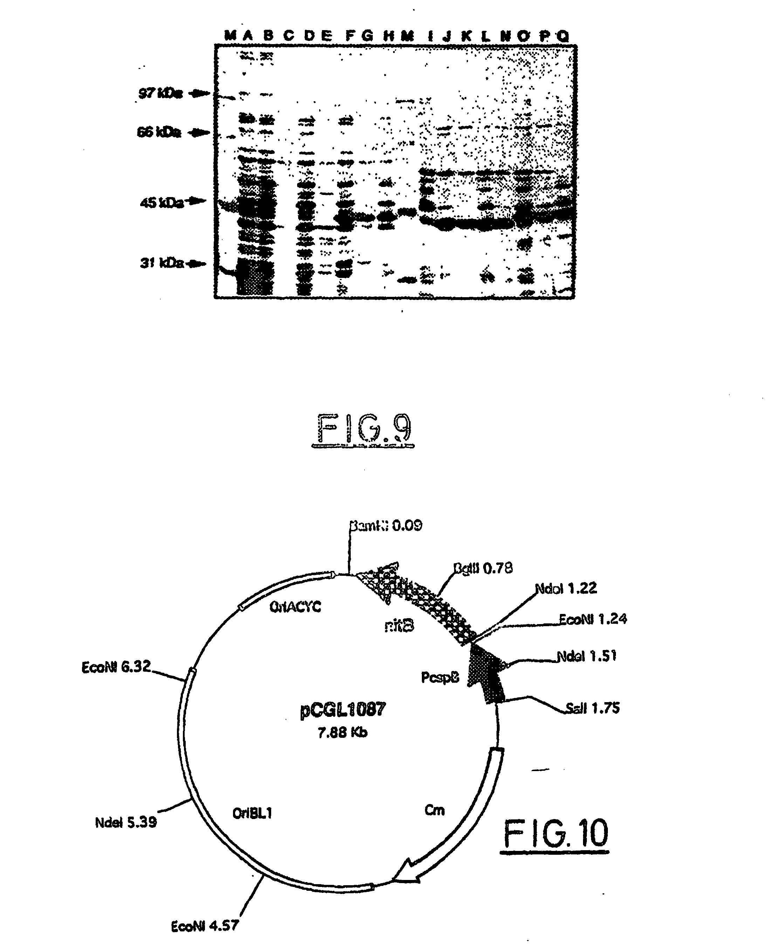

Après sonication des cellules, l'expression de la nitrilase a été mesurée sur un gel 10% SDS-PA dans la fraction brute après sonication des cellules, et après centrifugation dans le culot et dans le surnageant. Les résultats sont présentés sur la figure 9. Pour les souches RPA-BIOCAT1 (pKT230) et G2081 (pKT230), seuls les extraits bruts ont été déposés (pistes A et I respectivement). Sur cette figure, M représente le marqueur de poids moléculaires; ceux-ci sont indiqués en kDa. D'autre part, les pistes ont les significations ci-après :
- B, D, F représentent les fractions brutes obtenues respectivement à partir des souches RPA-BIOCAT1 (pRPA-BCAT14), RPA-BIOCAT1 (pRPA-BCAT23), RPA-BIOCAT1 (pRPA-BCAT24) ;
- C, E, G représentent les surnageants obtenus respectivement à partir de ces mêmes souches ;
- H représente le culot obtenus à partir de RPA-BIOCAT1 (pRPA-BCAT24) ;
- J, L, O représentent les fractions brutes obtenues respectivement à partir des souches G2081 (pRPA-BCAT14), G2081 (pRPA-BCAT23), G2081 (pRPA-BCAT24) ;
- K, N, P représentent les surnageants obtenus respectivement à partir de ces mêmes souches ;
- Q représente le culot obtenus à partir de G2081 (pRPA-BCAT24).

Cette expérience montre que les souches de *P. putida* expriment des quantités importantes des trois polypeptides nitrilasiques solubles et que seul le polypeptide nitrilasique à 44 kDa est surexprimé par la souche d'A. *faecalis*. Les dosages d'activité de ces cultures, réalisés selon le protocole décrit dans l'exemple 4, montrent que les souches G2081 (pRPA-BCAT23), G2081 (pRPA-BCAT24), et RPA-BIOCAT1 (pRPA-BCAT24) ont une activité nitrilasique sur l'HMTBN.

### Exemple 7 : Expression de la nitrilase dans Corynebacterium glutamicum.

- L'expression de la nitrilase a été réalisée dans la souche CGL1010 (= ATCC 14752) à l'aide du promoteur *PcspB* (Peyret *et al*., 1993, Molecular Microbiol. 9: 97-109). Un fragment de 530 bp contenant le promoteur P*cspB* a été amplifié à partir du plasmide pCGL815 (Peyret *et al*., précité) à l'aide des amorces KS1 et KS2 suivante:
KS1: 5'-ACGCGTCGACCAGATCGTCAAGTTGTGG-3'
KS2: 5'-CATAGAGGCGAAGGCTCCTTG-3'

Après digestion par *Sal* I, le fragment de 530 bp amplifié a été cloné dans le plasmide pBSK (Statagene, La Jolla USA) ouvert par *Sal* I et *Eco*RV pour donner le plasmide pCGL1084. Un adaptateur *Eco*NI/*Nde*I a été fabriqué en hybridant les deux oligonucléotides KS8 et KS9 suivants :
KS8: 5'-TCAAGGAGCCTTCGCCTCA-3'
KS9: 5'-TATGAGGCGAAGGCTCCTTG-3'

Le gène de la nitrilase a été extrait du plasmide pRPA-BCAT6 sous la forme d'un fragment *Nde*I*-Xba*I de 1,1 kb. Il a été introduit dans pCGL1084 ouvert avec *Eco*NI et *Xba*I en utilisant l'adaptateur *Eco*NI/*Nde*I décrit ci-dessus pour donner le plasmide pCGL1086. Le fragment *Sal*I-*Bam*HI de pCGL1086 contenant P*cspB*::*nitB* a ensuite été cloné dans le vecteur pCGL482 (Peyret *et al*., précité) aux sites *Sal* I et *Bam*HI conduisant au plasmide pCGL1087. Le plasmide pCGL1087 (figure **10**) est donc un vecteur navette à base de pBl1 (Santamaria *et al*., 1984, J. Gen. Microbiol. 130: 2237-2246) contenant l'origine de réplication de pACYC 184 reconnue chez *E. coli*, un gène conférant la résistance au chloramphenicol (Cm) et la fusion P*cspB::nitB*.

Les plasmides pCGL1087 et pCGL 482 ont été introduits par électroporation dans CGL1010 comme décrit par Bonnamy *et al*., 1990 (FEMS Microbiol. Lett. 66: 263-270). Après 20 heures de culture à 30°C en milieu Brain Heart Infusion 3,7% (Difco Laboratories, Detroit, USA) additionné de chloramphénicol 5 mg/l, des dosages d'activités nitrilase ont été réalisés sur des échantillons de culture comme décrit dans l'exemple 4. Les résultats montrent que la souche CGL1010 (pCGL1087) possède une activité nitrilase alors que la souche CGL1010 (pCGL482) n'en a pas.

### Exemple 8 : Expression de la nitrilase dans Streptomyces lividans.

L'expression de la nitrilase a été réalisée dans la souche de *S. lividans* TK24 (Hopwood *et al*., 1985, Genetic Manipulation of Streptomyces; A laboratory Manual, The John Innés Foundation, Norwich) à l'aide du plasmide plJ6021 (Takano *et al*., 1995, Gene 166: 133-137) en utilisant le promoteur P*tipA* (Holmes *et al*., 1993, EMBO J. 12: 3183-3191).

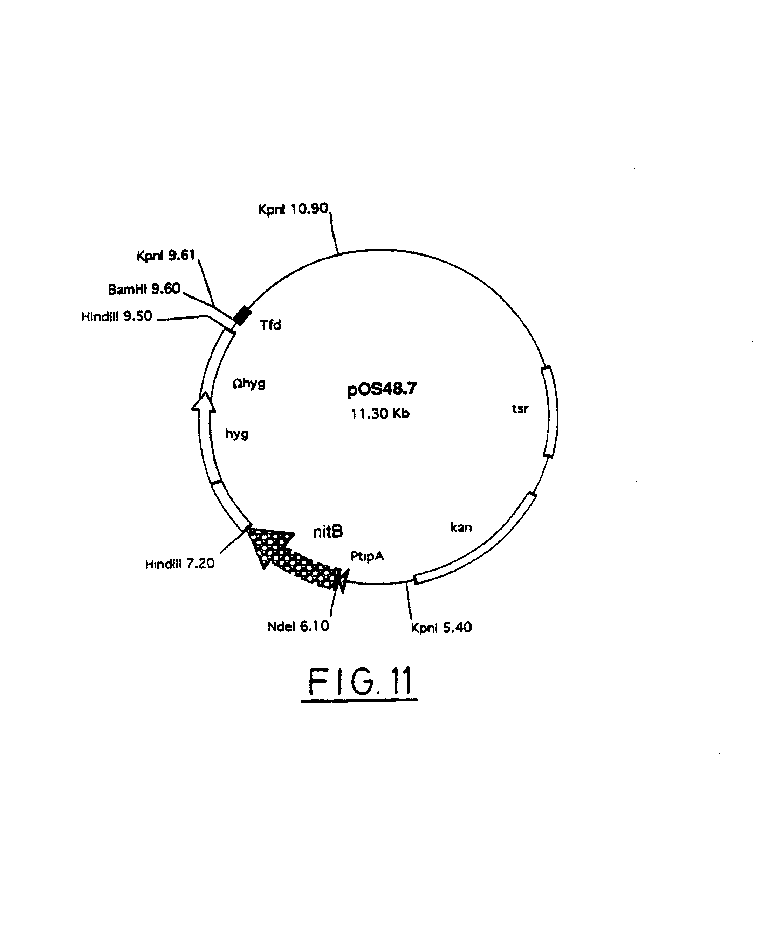

Le plasmide pUC19 (Yanisch-Perron *et al*., 1985, Gene 33: 103-119) a été modifié en délétant le fragment *Tfi* I de 140 bp par digestion *Tfi* I, traitement à la Klenow et ligature du vecteur sur lui-même. Le plasmide obtenu a été ouvert avec *Eco*RI et *Bam*HI afin de cloner le fragment de 1,15 kb *Eco*RI-*Bam*HI de pRPA-BCAT3 contenant le gène *nitB*. Le plasmide pOS48.4 ainsi obtenu possède un site unique *Tfi* I situé entre le gène *nitB* et le site *Bam*HI. Ce site a été utilisé pour insérer un fragment Ωhyg de 2,3 kb extrait du plasmide pHP45Ωhyg (Blondelet-Rouault *et al*., Gene, submitted) après digestion par *Bam*HI et traitement à la Klenow. Le fragment Ωhyg contient le gène de l'hygromycine phosphotransferase *(hyg)* de *Streptomyces hygroscopicus* (Zalacain *et al*., 1986, Nucl. Acids Res., 14: 1565-1581) et confère à *S. lividans* une resistance à l'hygromycine. Le plasmide pOS48.5 ainsi obtenu a été utilisé pour isoler un fragment *Nde*I-*Bam*HI de 3,45 kb contenant le gène de la nitrilase suivi du gène *hyg*, qui a été cloné dans le plasmide pIJ6021 (Takano *et al*., précité) ouvert par *Nde*I et *Bam*HI. Le plasmide plJ6021 ne se répliquant que dans *Streptomyces,* le mélange de ligation a été transformé dans *S. lividans* TK24 par des méthodes classiques (Hopwood *et al*., précité) en sélectionnant les clones résistant à 200 mg/l d'hygromycine (Boehringer Manheim). Les ADN plasmidiques de ces clones ont été extraits par des méthodes classiques (Hopwood *et al*., précité) et leurs profils de restriction correspondaient bien à la construction attendue qui a été appelée pOS48.7 (figure **11**).

Deux clones de *S. lividans* contenant pOS48.7 ainsi qu'un clone contenant le plasmide plJ6021 ont été cultivés dans 50 ml de milieu TSB (Tryptic Soy Broth, Difco) à 30 °C pendant 72 h avec une sélection kanamycine 5 mg/l, puis du thiostrepton a été ajouté à la concentration de 5 mg/l et la culture a été poursuivie pendant encore 18 h selon les conditions décrites (Hopwood *et al*., précité). La culture a été récoltée et le dosage d'activité, réalisé dans les conditions décrites dans l'exemple 4, montre que la souche *S. lividans* TK24 (pOS48.7) exprime une activité nitrilase contrairement à la souche TK24 (plJ6021).

### Exemple 9 : Hydrolyse de l'HMTBN avec d'autres nitrilases

La séquence primaire de la nitrilase de *Comamonas testosteroni sp*., décrite dans Levy-Schil *et al*., 1995 (précité) présente 31 % d'identité avec la séquence primaire de la nitrilase décrite dans cette invention. La souche recombinante d'*E*. *coli* TG1 (pXL2158, pXL2035), qui exprime la nitrilase de *C. testosteroni*, a été cultivée dans les conditions décrites par Lévy-Schil et *al*. (précité) et un culot cellulaire a été incubé dans du tampon phosphate 100 mM à pH 7 avec 50 mM HMTBN, à 30°C. L'activité mesurée était de 2,3 kg/h.kg CS. Comme la nitrilase de l'invention, la nitrilase de *C. testosteroni* est capable d'hydrolyser l'HMTBN.

De plus, les données présentées dans l'exemple 4 avec les plasmides pBCAT12 et pBCAT13 montrent que la substitution Asn279 -> Asp 279 sur la nitrilase d'*A. faecalis* ATCC8750 permet de conserver l'activité sur HMTBN.

### Exemple 10 : Apport du support

5 g de cellules sèches ont été ajoutés à 20 g d'eau ajustée à pH 7,0. La quantité de support (CELITE 545, Prolabo, France) indiquée est ensuite ajoutée et après une parfaite homogénéisation, la suspension est réticulée par ajout de 15 % de glutaraldéhyde basé sur la masse sèche totale suivi de 10 % de polyéthylèneimine (SEDIPUR, BASF, Allemagne). La suspension est agitée 1 heure à température ambiante.

La suspension est ensuite floculée par ajout de 0,001% d'un floculant anionique, Superfloc A100. La masse réticulée est récupérée par filtration.

Cette masse est ensuite de nouveau réticulée par ajout de 10% de polyazétidine (KYMENE 557, Hercules, USA) basé sur la masse sèche totale. La masse encore humide est ensuite extrudée à travers un orifice de 0,5 mm de diamètre et séchée.

L'activité des particules obtenues est ensuite déterminée suivant une procédure décrite dans le brevet WO 96/09403.

| g de support | Activité (en %/) |
|---|---|
| 0 | 100 |
| 2,5 g | 215 |
| 5 g | 250 |

L'ajout de support pour les cellules améliore notablement l'activité.

L'expérience a été répétée en substituant la CELITE par du gluten de blé (Roquette, France) partiellement soluble dans l'eau ou de la gélatine (SBI, France) totalement soluble dans l'eau.

| **Support** | **% activité** |
|---|---|
| 0 | 100 |
| 5 g de gluten | 160 |
| 5 g de gélatine | nd* |

| | |
|---|---|
| * La suspension cellulaire ne flocule pas et n'est pas filtrable. | |

Cet exemple montre que le gluten peut être substitué à la CELITE. En revanche, si l'ajout est de la gélatine (totalement soluble), la mise en forme du catalyseur est impossible par la technique décrite précédemment (extrusion).

### Exemple 11

10 g de *Escherichia coli* BIOCAT171 de l'exemple 4 préparés par culture aérobie dans un milieu LB ont été mélangés à 10 g de CLARCEL 78 (CECA; France) dans 500 g de tampon phosphate 100 mM pH 7,0. Après homogénéisation, 6 g de glutaraldéhyde 25% ont été ajoutés et la suspension a été agitée 15 minutes à température ambiante. 2 g de polyéthylènimine ont été ensuite ajoutés ainsi que 6 g de glutaraldéhyde à 25%. Le tout a été agité durant une heure à température ambiante. L'ensemble a été floculé par l'ajout de 5 ml d'une solution de SUPERFLOC A100 à 0,2 % (CYTEC, France). La masse a été filtrée et la pâte obtenue a été mélangée avec 16 g de polyazétidine à 12,5% (KYMENE 557, Hercules) puis extrudée à travers un orifice de 0,5 mm de diamètre. Les vermicelles obtenus ont été séchés à 35°C dans une étuve puis plongés 30 minutes dans un bain de NaBH₄ à 1% préparé dans un tampon borate 50 mM pH 10,0. Le biocatalyseur est ensuite lavé à l'eau distillée. Le catalyseur est conservé à 5°C ou à température ambiante dans un tampon phosphate 500 mM pH 8,0.

Une colonne thermostatée de 3 cm de diamètre intérieur et de 45 cm de haut a été remplie avec 100 g de biocatalyseur. Cette colonne est reliée à une pompe via une boucle de recirculation. Le volume total du réacteur est de 430 ml. La boucle est remplie d'une solution de sel d'ammonium de l'acide hydroxy méthylthio butyrique à 25%. La solution est alimentée par le haut de la colonne vers le bas à un débit horaire de 20 l/h. L'eau qui circule dans la double enveloppe de la colonne et dans l'échangeur thermique permet de maintenir la température à 35°C. L'eau déminéralisée est ajoutée à la boucle à un débit de 80 g/h. Le 2-hydroxy 4-méthylthiobutyronitrile est ajouté à 20 g/ h. L'excédent de volume du milieu de réaction est évacué par le bas de la colonne de telle manière que le volume de la boucle reste constant. Ainsi, on obtient un flux continu avec un taux de transformation du nitrile de 95 %. De plus la concentration de la solution aqueuse de sel d'ammonium de l'acide 2-hydroxy 4-méthylthio butyrique obtenue en sortie du réacteur est de 25%.

### Exemple 12

L'électrodialyseur utilisé est constitué d'un empilement de 9 cellules de 2 dm² de surface active, composées chacune d'elles de 2 compartiments désignés comme suit :
- compartiment sel/acide : limité du côté cathode par une membrane échangeuse de cations Neosepta CMB de Tokuyama Soda, et du côté anode par la face cationique d'une membrane bipolaire Aqualytics
- compartiment base : limité du côté anode par la membrane cationique, et du côté cathode par la face anionique de la membrane bipolaire.

L'anode est constituée de titane platiné. La cathode est en acier inoxydable.

L'électrolyte est constitué par une solution aqueuse de sulfate de sodium ayant une conductivité de 100 mS/cm à 40°C. Le débit de circulation aux électrodes est de 2x100 l/h.Le volume est de 5 l.

Le compartiment "base" est initialement rempli de 5 litres d'une solution de sulfate d'ammonium à 1 %.

Le compartiment "sel/acide" est initialement rempli de 5 litres d'une solution à 1,44 mole/l du sel d'ammunium de l'acide 2-hydroxy-4-(méthylthio)butanoïque.

Le débit de recirculation des solutions est initialement fixé à 130 l/h pour le compartiment sel/acide et à 190 l/h pour le compartiment base.

L'électrodialyse est effectuée en mode discontinu (fonctionnement en recirculation), à une température moyenne de 40°C. L'intensité est imposée à 9 A, soit une densité de courant de 0,45 kA/m².

Après 155 minutes de fonctionnement, la conductivité du compartiment sel/acide est passée de 59 à 7,8 mS/cm.

Le compartiment sel/acide contient 1,35 mole/l d'acide 2-hydroxy-4-(méthylthio)butanoïque, à 100% sous forme acide.

Le rendement faradique est estimé à 71% et la consommation énergétique à 0,53 kWh par kg d'acide formé.

### Exemple 13

L'électrodialyseur utilisé est constitué d'un empilement de 8 cellules de configuration identique à celle de l'exemple 12.

Le compartiment "base" est initialement rempli de 5,27 litres d'une solution de sulfate d'ammonium à 1%.

Le compartiment "sel/acide" est initialement rempli de 4,85 litres d'une solution à 1,39 mole/l du sel d'ammonium de l'acide 2-hydroxy-4-(méthylthio)butanoïque.

Le débit de recirculation des solutions est initialement fixé à 60 l/h pour le compartiment sel/acide et à 150 l/h pour le compartiment base.

L'électrodialyse est effectuée en mode discontinu (fonctionnement en recirculation), à une température moyenne de 40°C. L'intensité est imposée à 9 A, soit une densité de courant de 0,45 kA/m².

Après 172 minutes de fonctionnement, la conductivité du compartiment sel/acide est passée de 59 à 9,5 mS/cm.

Le volume final du compartiment sel/acide est de 4,67 litres.

La composition est la suivante:

| | |
|---|---|
| acide 2-hydroxy-4-(méthylthio)butanoïque: | 1,24 mole/l |
| 2-hydroxy-4-(méthylthio)butanoate d'ammonium: mole/I | 0,148 |

Le taux de transformation est de 86 %. Le produit final est à 89 % sous forme acide.

Le rendement faradique est de 74%. La consommation énergétique est estimée à 0,58 kWh par kg d'acide formé.

### Exemple 14

On utilise un dispositif analogue à celui de l'exemple 13.

Le compartiment "base" est initialement rempli de 5,46 litres d'une solution de sulfate d'ammonium à 1%.

Le compartiment "sel/acide" est initialement rempli de 5,23 litres d'une solution à 1,24 mole/l du sel d'ammonium de l'acide 2-hydroxy-4-(méthylthio)butanoïque.

Le débit de recirculation des solutions est initialement fixé à 90 l/h pour le compartiment sel/acide et à 150 l/h pour le compartiment base.

L'électrodialyse est effectuée en mode discontinu (fonctionnement en recirculation), à une température moyenne de 40°C. L'intensité est imposée à 14 A, soit une densité de courant de 0,7 kA/m².

Après 105 minutes de fonctionnement, la conductivité du compartiment sel/acide est passée de 57,6 à 9,8 mS/cm.

La composition finale du compartiment sel/acide est la suivante:

| | |
|---|---|
| acide 2-hydroxy-4-(méthylthio)butanoïque | 1,28 mole/l |
| 2-hydroxy-4-(méthylthio)butanoate d'ammonium | 0,14 mole/l |

Le produit est donc à 90% sous forme acide.

### Exemple 15

On utilise un dispositif analogue à celui de l'exemple 13.

L'essai est mis en oeuvre avec le contenu du compartiment base obtenu à l'issue de l'exemple 14.

Le compartiment "sel/acide" est initialement rempli de 5,2 litres d'une solution à 1,44 mole/l du sel d'ammonium de l'acide 2-hydroxy-4-(méthylthio)butanoïque.

Le débit de recirculation des solutions est initialement fixé à 50 l/h pour le compartiment sel/acide et à 150 I/h pour le compartiment base.

L'électrodialyse est effectuée en mode discontinu (fonctionnement en recirculation), à une température moyenne de 42°C. L'intensité est imposée à 19 A, soit une densité de courant de 0,95 kA/m².

Après 53 minutes de fonctionnement, la conductivité du compartiment sel/acide est passée de 59 à 27 mS/cm.

Le volume final du compartiment sel/acide est de 4,85 litres.

La composition est la suivante:

| | |
|---|---|
| acide 2-hydroxy-4-(méthylthio)butanoïque | 0,87 mole/l |
| 2-hydroxy-4-(méthylthio)butanoate d'ammonium | 0,56 mole/l |

Le taux de transformation est de 56%. Le produit final est à 61% sous forme acide.

Le rendement faradique est de 83%. La consommation énergétique est estimée à 0,7 kWh par kg d'acide formé.

### Exemple 16

On concentre en batch 200,1 g de solution d'HMTBS à environ 1,5 M dans un évaporateur rotatif. La température du bain est de 45+/- 5 °C. La pression est régulée aux alentours de 2,5.10³ Pa (25 mbar). La température dans le bouilleur évolue de 25°C en début de distillation jusqu'à 40°C en fin. Au bout de 3 heures, on récupère 41,9 g d'un milieu visqueux jaune dont les caractéristiques sont les suivantes :

| | |
|---|---|
| viscosité à 25°C (mm².s⁻¹) | 1150 |
| % HMTBA (Br-/BrO3-) | 83,3 |

Après ajustement du titre (dilution à H₂O), on obtient un produit dont les caractéristiques sont les suivantes :

| | |
|---|---|
| viscosité à 25°C (mm².s⁻¹) | 396 |
| % HMTBA (Br-/BrO3-) | 79,5 |

Par potentiométrie on a la répartition massique suivante :

| | |
|---|---|
| % HMTBS | 81,6 |
| % HMTBA | 6,0 |

En CLHP, on mesure le rapport des surfaces monomère ou dimères/(monomère+diméres)

| | |
|---|---|
| monomère/(monomère+dimères) | 100 |
| dimères/(monomère+dimères) (*) | 0 |

| | |
|---|---|
| (*) on ne détecte pas de dimères | |

### Exemple 17

On concentre en batch 200.0 g de solution d'HMTBS à environ 1,5 M dans un évaporateur rotatif. La température du bain est de 121+/- 5 °C. La pression est régulée aux alentours de 9,5.10⁴ Pa. La température dans le bouilleur évolue de 100°C en début de distillation jusqu'à 113°C en fin. Au bout de 3 heures, on récupère 41,5 g d'un milieu visqueux brun dont les caractéristiques sont les suivantes :

| | |
|---|---|
| viscosité à 25°C (mm².s⁻¹) | - |
| % HMTBA (Br-/BrO3-) | 90,7 |

Après ajustement du titre (dilution à H₂O), on obtient un produit dont les caractéristiques sont les suivantes :

| | |
|---|---|
| viscosité à 25°C (mm².s⁻¹) | 117 |
| % HMTBA (Br-/BrO3-) | 78,8 |

Par potentiométrie, on a la répartition massique suivante :

| | |
|---|---|
| % HMTBS | 52,2 |
| % HMTBA | 28,7 |

En CLHP, on mesure le rapport des surfaces monomère ou dimères/(dimères+monomère)

| | |
|---|---|
| monomère/(monomère+dimères) | 95,0 |
| dimères/(monomère+dimères) | 5,0 |

### Exemple 18

A 100,0 g du milieu décrit dans l'exemple n°17, on rajoute 40,0 g de H₂O . On extrait le milieu par 75,0 g d'éther isopropylique. Après séparation des phases, on récupère dans la phase organique après évaporation, 25,6 g d'HMTBA dont les caractéristiques sont les suivantes :

| | |
|---|---|
| détermination | |
| monomère/(monomère+dimères) (% surface) | 97,0 |
| dimères/(monomère+dimères) (% surface) | 3,0 |
| viscosité à 25 °C (mm².s⁻¹) | 55 |

### Exemple 19 :Vieillissement des solutions décrites dans les exemples précédents

| | exemple n°16 | exemple n°17 | exemple n°18 |
|---|---|---|---|
| Durée de stockage à 25°C | 40 j | 40 j | 100 j |
| viscosité (mm².s⁻¹) | 387 | 116 | 66 |
| | | | |
| monomère/(monomère+dimère) | 100,0 | 95,0 | 82 |
| dimères/(monomère+dimères) | 0,0 | 5,0 | 18 |

Les solutions des exemples 16 et 17 n'ont pas évolué dans leur tenue en dimères après 40 jours de stockage.

## Revendications

1. Procédé en continu de préparation de l'acide 2-hydroxy 4-méthylthio butyrique et/ou du sel d'ammonium de l'acide 2-hydroxy 4-méthylthio butyrique par hydrolyse enzymatique du 2-hydroxy 4-méthylthio butyronitrile susceptible d'application industrielle **caractérisé en ce que** :
a) dans une première étape on prépare un matériel biologique ayant une activité nitrilase
b) dans une deuxième étape, on l'immobilise,
c) dans une troisième étape, on met en présence le 2-hydroxy 4-méthylthio butyronitrile avec le matériel biologique ainsi immobilisé, pour obtenir le sel d'ammonium de l'acide 2-hydroxy 4-méthylthio butyrique,
d) dans une quatrième étape, éventuellement, on convertit le sel obtenu à l'étape c) en l'acide correspondant, et
e) dans une cinquième étape, on concentre le produit obtenu à l'étape c) ou d).

2. Procédé selon la revendication 1, **caractérisé en ce que** l'activité nitrilase est obtenue à partir d'une nitrilase d'*Alcaligenes*, de préférence *faecalis*.

3. Procédé selon la revendication 1 ou la revendication 2, **caractérisé en ce que** l'on utilise l'information génétique codant pour une nitrilase que l'on exprime dans un microorganisme hôte.

4. Procédé selon la revendication 3, **caractérisé en ce que** le microorganisme hôte est choisi parmi *Escherichia coli* ou un membre du genre *Bacillus, Corynebacterium, Streptomyces, Saccharomyces, Kluyveromyces, Penicillium* et *Aspergillus*.

5. Procédé selon la revendication 1, **caractérisé en ce que** l'activité nitrilase est obtenue à partir de la nitrilase codée par le gène cloné dans le plasmide pRPA-BCAT3, déposé à la CBS sous le numéro CBS 998-96.

6. Procédé selon l'une quelconque des revendications 3 à 5, **caractérisé en ce que** la nitrilase est co-exprimée avec une protéine chaperonne.

7. Procédé selon la revendication 6, **caractérisé en ce que** la protéine chaperonne est GroESL dans *E. coli*.

8. Procédé selon l'une quelconque des revendications précédentes, **caractérisé en ce que** le matériel biologique de l'étape a) est immobilisé sur un support solide.

9. Procédé selon la revendication 8, **caractérisé en ce que** le support solide a une granulométrie comprise entre 1 µm et 3 mm, de préférence 10 µm et 2 mm, et **en ce qu'**il est ajouté à raison de 0,01 à 500 %, de préférence 10 à 200 % en poids du matériel biologique.

10. Procédé selon la revendication 9, en ce que le support solide est choisi parmi :
les résines échangeuses d'ions,
l'alumine,
les silice de synthèse ou diatomées et les gels de silice,
les zéolites,
les charbons,
les protéines partiellements solubles dans l'eau, et
les polysaccharides.

11. Procédé selon la revendication 10, **caractérisé en ce que** le support solide est le gluten.

12. Procédé selon la revendication 8, **caractérisé en ce que** l'on utilise un ou plusieurs agents chimiques pour réticuler et/ ou insolubiliser le matériel biologique et le support.

13. Procédé selon la revendication 12, **caractérisé en ce que** les agents chimiques sont choisis parmi :
les polymères de polyazétidine,
les polymères de polyéthylènimine,
les polymères de polyamides,
les polymères d'isocyanates,
les gels d'alginate,
les gels de k-carraghénane,
les amines,
les aldéhydes,
les acides carboxyliques, et
les isocyanates.

14. Procédé selon les revendications 10 à 13, **caractérisé en ce que** le matériel biologique et le support sont mélangés en présence d'agents chimiques pour obtenir une pâte qui est extrudée puis séchée.

15. Procédé selon les revendications 10 à 13, **caractérisé en ce que** le matériel biologique et les agents chimiques sont mélangés puis déposés sur le support sous forme d'une couche mince.

16. Procédé selon l'une quelconque des revendications précédentes, **caractérisé en ce qu'**on obtient un mélange du sel d'ammonium de l'acide 2-hydroxy 4-méthylthio butyrique et d' l'acide 2-hydroxy 4-méthylthio butyrique contenant au moins 60% d' l'acide 2-hydroxy 4-méthylthio butyrique, et de préférence au moins 80% d' l'acide 2-hydroxy 4-méthylthio butyrique, le complément étant du sel d'ammonium de l'acide 2-hydroxy 4-méthylthio butyrique, par dissociation électrolytique du sel d'ammonium correspondant.

17. Procédé selon la revendication 16, **caractérisé en ce que** la dissociation est réalisée dans un électrodyaliseur à membranes bipolaires à trois compartiments.

18. Procédé selon la revendication 16, **caractérisé en ce que** la dissociation est réalisée dans un électrodyaliseur à membranes bipolaires à deux compartiments.

19. Procédé selon l'une quelconque des revendications 1 à 15, **caractérisé en ce qu'**un mélange du sel d'ammonium de l'acide 2-hydroxy 4-méthylthio butyrique et d' l'acide 2-hydroxy 4-méthylthio butyrique est obtenu par chauffage d'une solution de sel d'ammonium.

20. Procédé selon les revendications 16 à 19, **caractérisé en ce que** l'ammoniaque libérée est distillée, concentrée et recyclée dans la synthèse d'HCN.

21. Solution aqueuse obtenue selon les revendications 16 à 19 constituée d'un mélange d' l'acide 2-hydroxy 4-méthylthio butyrique et du sel d'ammonium de l'acide 2-hydroxy 4-méthylthio butyrique dont le rapport pondéral acide/ (acide+sel) est de 5 à 99,9% et de préférence de 10 à 50%.

22. Installation pour la mise en oeuvre du procédé selon l'invention, comprenant :
- un ou plusieurs réacteurs (3,4) garni avec des enzymes immobilisées ayant une activité nitrilase, ou des microorganismes hôtes immobilisés, exprimant une enzyme à activité nitrilase,
- éventuellement, un ou plusieurs moyens d'électrodialyse (9),
- des moyens de concentration (6,12) du produit final.

23. Plasmide pRPA-BCAT3, déposé à la CBS sous le numéro CBS 998-96.

## Claims

1. Continuous process for the preparation of 2-hydroxy-4-methylthiobutyric acid and/or of the ammonium salt of 2-hydroxy-4-methylthio butyric acid by enzymatic hydrolysis of 2-hydroxy-4-methylthio-butyronitrile which is capable of being applied industrially, **characterized in that**:
a) in a first stage, a biological material having a nitrilase activity is prepared,
b) in a second stage, it is immobilized,
c) in a third stage, the 2-hydroxy-4-methylthiobutyronitrile is exposed to the biological material thus immobilized, in order to obtain the ammonium salt of 2-hydroxy-4-methylthiobutyric acid,
d) in a fourth stage, optionally, the salt obtained in stage c) is converted to the corresponding acid, and
e) in a fifth stage, the product obtained in stage c) or d) is concentrated.

2. Process according to claim 1, **characterized in that** the nitrilase activity is obtained from an *Alcaligenes*, preferably *faecalis*, nitrilase.

3. Process according to claim 1 or claim 2, **characterized in that** the genetic information encoding a nitrilase is used which is expressed in a host microorganism.

4. Process according to claim 3, **characterized in that** the host microorganism is chosen from *Escherichia coli* or a member of the genus *Bacillus, Corynebacterium, Streptomyces, Saccharomyces, Kluyveromyces, Penicillium* and *Aspergillus*.

5. Process according to claim 1, **characterized in that** the nitrilase activity is obtained from the nitrilase encoded by the gene cloned into the plasmid pRPA-BCAT3, deposited at CBS under the number CBS 998-96.

6. Process according to any one of claims 3 to 5, **characterized in that** the nitrilase is coexpressed with a chaperon protein.

7. Process according to claim 6, **characterized in that** the chaperon protein is GroESL in *E. coli*.

8. Process according to any one of the preceding claims, **characterized in that** the biological material of the stage a) is immobilized on a solid support.

9. Process according to claim 8, **characterized in that** the solid support has a particle size of between 1 µm and 3 mm, preferably 10 µm and 2 mm, and **in that** it is added in an amount of 0.01 to 500%, preferably 10 to 200% by weight of the biological material.

10. Process according to claim 9, **characterized in that** the solid support is chosen from:
ion-exchange resins,
alumina,
synthetic silicas or diatomaceous earth and
silica gels,
zeolites,
charcoals,
partially water-soluble proteins, and
polysaccharides.

11. Process according to claim 10, **characterized in that** the solid support is gluten.

12. Process according to claim 8, **characterized in that** one or more chemical agents are used to cross-link and/or insolubilize the biological material and the support.

13. Process according to claim 12, **characterized in that** the chemical agents are chosen from:
polyazetidine polymers,
polyethyleneimine polymers,
polyamide polymers,
isocyanate polymers,
alginate gels,
k-carrageenan gels,
amines,
aldehydes,
carboxylic acids, and
isocyanates.

14. Process according to claims 10 to 13, **characterized in that** the biological material and the support are mixed in the presence of chemical agents in order to obtain a paste which is extruded and then dried.

15. Process according to claims 10 to 13, **characterized in that** the biological material and the chemical agents are mixed and then deposited on the support in the form of a thin layer.

16. Process according to any one of the preceding claims, **characterized in that** a mixture of the ammonium salt of 2-hydroxy-4-methylthiobutyric acid and 2-hydroxy-4-methylthiobutyric acid containing at least 60% 2-hydroxy-4-methylthiobutyric acid, and preferably at least 80% 2-hydroxy-4-methylthiobutyric acid, the balance being ammonium salt of 2-hydroxy-4-methylthiobutyric acid, is obtained by electrolytic dissociation of the corresponding ammonium salt.

17. Process according to claim 16, **characterized in that** the dissociation is carried out in an electrodialyser with bipolar membranes having three compartments.

18. Process according to claim 16, **characterized in that** the dissociation is performed in an electrodialyser with bipolar membranes having two compartments.

19. Process according to any one of claims 1 to 15, **characterized in that** a mixture of the ammonium salt of 2-hydroxy-4-methylthiobutyric acid and 2-hydroxy-4-methylthiobutyric acid is obtained by heating an ammonium salt solution.

20. Process according to claims 16 to 19, **characterized in that** the liberated ammonium hydroxide is distilled, concentrated and recycled in the HCN synthesis.

21. Aqueous solution obtained according to claims 16 to 19, consisting of a mixture of 2-hydroxy-4-methylthiobutyric acid and the ammonium salt of 2-hydroxy-4-methylthiobutyric acid whose acid/(acid+salt) weight ratio is 5 to 99.9% and preferably 10 to 50%.

22. Plant for carrying out the process according to the invention comprising:
- one or more reactors (3, 4) packed with immobilized enzymes having a nitrilase activity, or with immobilized host microorganisms expressing an enzyme with nitrilase activity,
- optionally, one or more electrodialysis means (9),
- means for concentration (6, 12) of the final product.

23. Plasmid pRPA-BCAT3, deposited at CBS under the number CBS 998-96.

## Patentansprüche

1. Kontinuierliches Verfahren zur Herstellung von 2-Hydroxy-4-methylthiobuttersäure und/oder dem Ammoniumsalz von 2-Hydroxy-4-methylthiobuttersäure durch enzymatische Hydrolyse von 2-Hydroxy-4-methylthiobutyronitril, das sich für die industrielle Anwendung eignet, **dadurch gekennzeichnet, dass**
a) in einem ersten Schritt ein biologisches Material hergestellt wird, das eine Nitrilaseaktivität aufweist,
b) in einem zweiten Schritt dieses immobilisiert wird,
c) in einem dritten Schritt das 2-Hydroxy-4-methylthiobutyrontril mit dem so immobilisierten biologischen Material, zum Erhalt des Ammoniumsalzes von 2-Hydroxy-4-methylthiobuttersäure in Kontakt gebracht wird,
d) in einem vierten Schritt, gegebenenfalls, das in schritt c) erhaltene Salz in die korrespondierende Säure umgewandelt wird, und
e) in einem fünften Schritt das in Schritt c) oder in Schritt d) erhaltene Produkt konzentriert wird.

2. Verfahren nach Anspruch 1, **dadurch gekennzeichnet, dass** die Nitrilaseaktivität ausgehend von einer Nitrilase aus *Alcaligenes*, vorzugsweise *faecalls*, erhalten wird.

3. Verfahren nach Anspruch 1 oder 2, **dadurch gekennzeichnet, dass** die für eine Nitrilase codierende genetische Information, die in einem Wirtsmikroorganismus exprimiert wird, verwendet wird.

4. Verfahren nach Anspruch 3, **dadurch gekennzeichnet, dass** der Wirtsmikroorganismus ausgewählt ist aus *Escherichia coli* oder einem Mitglied der Gattung *Bazillus, Corynebacterium, Streptomyces, Saccharomyces, Kluyveromyces, Penicillium* und *Aspergillus*.

5. Verfahren nach Anspruch 1, **dadurch gekennzeichnet, dass** die Nitrilaseaktivität ausgehend von der Nitrilase erhalten wird, die durch das in dem Plasmid pRPA-BCAT3 klonierte Gen codiert wird, das bei der CBS unter der Nummer CBS 998-96 hinterlegt ist.

6. Verfahren nach einem der Ansprüche 3 bis 5, **dadurch gekennzeichnet, dass** die Nitrilase mit einem Chaperonprotein koexprimiert wird.

7. Verfahren nach Anspruch 6, **dadurch gekennzeichnet, dass** das Chaperonprotein in *E. coli* GroESL ist.

8. Verfahren nach einem der vorhergehenden Ansprüche, **dadurch gekennzeichnet, dass** das biologische Material des Schritts a) an einem festen Träger immobilisiert ist.

9. Verfahren nach Anspruch 8, **dadurch gekennzeichnet, dass** der feste Träger eine Korngröße zwischen 1 µm und 3 mm, vorzugsweise zwischen 10 µm und 2 mm aufweist, und dass er dem biologischen Material in einem Verhältnis von 0,01 bis 500 Gew.-%, bevorzugt 10 bis 200 Gew.-% zugesetzt wird.

10. Verfahren nach Anspruch 9, **dadurch gekennzeichnet, dass** der feste Träger ausgewählt ist aus:
Ionentauscherharzen,
Aluminiumoxid,
synthetischer Kieselerde oder Diatomeenerde und
Silikagelen,
Zeoliten,
Aktivkohlen,
teilweise in Wasser löslichen Proteinen und
Polysacchariden.

11. Verfahren nach Anspruch 10, **dadurch gekennzeichnet, dass** der feste Träger Gluten ist.

12. Verfahren nach Anspruch 8, **dadurch gekennzeichnet, dass** ein oder mehrere chemische Mittel verwendet werden, um das biologische Material und den Träger zu vernetzen und/oder unlöslich zu machen.

13. Verfahren nach Anspruch 12, **dadurch gekennzeichnet, dass** die chemischen Mittel ausgewählt sind aus:
Polyazetidinpolymeren,
Polyethyleniminpolymeren,
Polyamidpolymeren,
Isocyanatpolymeren,
Alginatgelen,
K-Carraghenangelen,
Aminen,
Aldehyden,
Carbonsäuren, und
Isocyanaten.

14. Verfahren nach einem der Ansprüche 10 bis 13, **dadurch gekennzeichnet, dass** das biologische Material und der Träger in Gegenwart der chemischen Mittel gemischt werden, um eine Paste zu erhalten, die extrudiert und dann getrocknet wird.

15. Verfahren nach einem der Ansprüche 10 bis 13, **dadurch gekennzeichnet, dass** das biologische Material und die chemischen Mittel gemischt werden und dann auf den Träger in Form einer dünnen Schicht aufgetragen werden.

16. Verfahren nach einem der vorhergehenden Ansprüche, **dadurch gekennzeichnet, dass** durch elektrolytische Dissoziation des entsprechenden Ammoniumsalzes eine Mischung des Ammoniumsalzes von 2-Hydroxy-4-methylthiobuttersäure und der 2-Hydroxy-4-methylthiobuttersäure erhalten wird, die zumindest 60 % 2-Hydroxy-4-methylthiobuttersäure und bevorzugt zumindest 80 % der 2-Hydroxy-4-methylthiobuttersäure aufweist, wobei der Rest aus dem Ammoniumsalz von 2-Hydroxy-4-methylthiobuttersäure besteht.

17. Verfahren nach Anspruch 16, **dadurch gekennzeichnet, dass** die Dissoziation in einem Elektrodialysegerät mit bipolaren Membranen, das drei Kammern aufweist, durchgeführt wird.

18. Verfahren nach Anspruch 16, **dadurch gekennzeichnet, dass** die Dissoziation in einem Elektrodialysegerät mit bipolaren Membranen, das zwei Kammern aufweist, durchgeführt wird.

19. Verfahren nach einem der Ansprüche 1 bis 15, **dadurch gekennzeichnet, dass** die Mischung aus dem Ammoniumsalz von 2-Hydroxy-4-methylthiobuttersäure und der 2-Hydroxy-4-methylthiobuttersäure durch Erwärmen einer Lösung des Ammoniumsalzes erhalten wird.

20. Verfahren nach einem der Ansprüche 16 bis 19, **dadurch gekennzeichnet, dass** der freigesetzte Ammoniak destilliert, konzentriert und bei der HCN-Synthese wiederverwendet wird.

21. Wässrige Lösung, die mit dem Verfahren nach einem der Ansprüche 16 bis 19 erhalten wurde, die aus einer Mischung der 2-Hydroxy-4-methylthiobuttersäure und des Ammoniumsalzes von 2-Hydroxy-4-methylthiobuttersäure besteht, wobei das Gewichtsverhältnis Säure/(Säure + Salz) zwischen 5 und 99,9 % und vorzugsweise zwischen 10 und 50 % liegt.

22. Vorrichtung zur Durchführung des erfindungsgemäßen Verfahrens, die Folgendes aufweist:
- einen oder mehrere Reaktoren (3, 4), die mit immobilisierten Enzymen, die eine Nitrilaseaktivität aufweisen, oder immobilisierten Wirtsmikroorganismen versehen sind, die ein Enzym mit Nitrilaseaktivität exprimieren,
- gegebenenfalls ein oder mehrere Mittel zur Elektrodialyse (9),
- Mittel zum Konzentrieren (6, 12) des fertigen Produkts.

23. Plasmid pRPA-BCAT3, hinterlegt bei der CBS unter der Nummer CBS 998-96.
